# EUROPEAN PATENT APPLICATION

(11) **EP 2 317 316 A2**
(43) Date of publication of application: **04.05.2011**
(21) Application number: 10015900.3
(22) Date of filing: 07.07.2006
(51) Int. Cl.: G01N 33/50, G01N 33/94

(54) **Methods for identifying agent and conditions that modulate neurogenesis**

(30) Priority: 08.07.2005 US 697905 P; 31.01.2006 US 763883 P
(62) Divisional of application: 06800028.0
(71) Applicant: Braincells, Inc., San Diego, CA 92121 (US)
(72) Inventor: Barlow, Carrolee, Del Mar, California 92014 (US); Pires, Jammieson, San Diego, California 92139 (US); Lorrain, Kym I., San Diego, California 92124 (US); Carter, Todd, San Diego, California 92129 (US); Treuner, Kai, San Diego, California 92122 (US)
(74) Representative: Roques, Sarah Elizabeth

(57) **Abstract**

Methods and tools for identifying agents and conditions that modulate neurogenesis are disclosed. The disclosure also relates to methods and tools for identifying populations of neural stem cells suitable for transplantation.

## Description

### CROSS-REFERENCES TO RELATED APPLICATIONS

This application claims benefit of priority under 35 U.S.C. § 119(e) from U.S. Provisional Patent Applications 60/697,905, filed July 8, 2005, and 60/763,883, filed January 31, 2006, both of which are hereby incorporated by reference as if fally set forth.

### FIELD OF THE DISCLOSED INVENTION

The disclosed invention relates to methods and tools for identifying agents and conditions that modulate neurogenesis. Moreover, the disclosed invention relates to methods and compositions relating to the *ex vivo* preparation of neural cells for transplantation into a subject. The disclosed invention also relates to methods and tools for identifying populations of neural stem cells suitable for transplantation.

### BACKGROUND

Neurogenesis is a vital process in the brains of animals and humans, whereby new nerve cells are continuously generated throughout the life span of the organism. The newly generated cells are able to differentiate into functional cells of the central nervous system and integrate into existing neural circuits in the brain. Neurogenesis persists throughout adulthood in two restricted regions of the mammalian brain: the subventricular zone (SVZ) of the lateral ventricles and the dentate gyrus of the hippocampus. In these regions, multipotent neural progenitor cells (NPCs) continue to divide and give rise to new functional neurons and glial cells (for review Gage 2000). It has been shown that a variety of factors can stimulate adult hippocampal neurogenesis, e.g. adrenalectomy, voluntary exercise, enriched environment, hippocampus dependent learning and anti-depressants (Yehuda 1989, van Praag 1999, Brown J 2003, Gould 1999, Malberg 2000, Santarelli 2003). Other factors, such as adrenal hormones, stress, age and drugs of abuse negatively influence neurogenesis (Cameron 1994, McEwen 1999, Kuhn 1996, Eisch 2004).

Drugs with the potential to modulate neurogenesis hold great promise as therapeutic agents against many diseases-including, but not limited to, Alzheimer's disease, Parkinson's disease, traumatic brain injury, developmental disorders, depression and mood disorders, stroke, and epilepsy. For example, Parkinson's disease is a progressive neurodegenerative disorder characterized by the loss of the nigrostriatal pathway as a result of degeneration of dopaminergic neurons within the substantia nigra. Although the cause of Parkinson's disease is not known, it is associated with the progressive death of dopaminergic (tyrosine hydroxylase (TH) positive) mesencephalic neurons, inducing motor impairment. The characteristic symptoms of Parkinson's disease appear when up to 70% of TH-positive nigrostriatal neurons have degenerated. Surgical therapies aimed at replacing lost dopaminergic neurons or disrupting aberrant basal ganglia circuitry have recently been tested (C. Honey et al. 1999), but the primary goal of forestalling disease progression in newly diagnosed patients has yet to be realized. Thus, there are currently no satisfactory methods for curing, preventing or treating Parkinson's disease or its symptoms. However, considering the role of neurodegeneration in Parkinson's disease, neurogenesis-based treatments provide a means for directly treating the underlying cause of the disease.

Agents that modulate neurogenesis also hold promise for the treatment of depression and other mood disorders. For example, neurogenesis is thought to play an important homeostatic role in the hippocampus of depressed patients. Pathological stimuli, such as depression, can cause neuronal atrophy and death, which leads to a reduction in hippocampal volume that is correlated with the length and severity of the depression. Antidepressant medications have been reported as able to reverse the reduction in hippocampal volume. Known antidepressants have been reported in animal models as exhibiting such a stimulatory effect, and genetic models suggest that hippocampal neurogenesis may be required for antidepressant activity. For example, neurogenesis has been reported pre-clinically to be required for the antidepressant efficacy of Prozac and other antidepressant drugs (Santarelli, Saxe et al. 2003). Moreover, the time required for the therapeutic onset of action of antidepressants has been reported to correlate with the time course of neurogenesis. Thus, evidence suggests that the ability of currently available antidepressant medications to treat depression is at least partly due to their neurogenesis-stimulating properties.

Despite their effects on neurogenesis, most currently available antidepressants were primarily developed to modulate other processes. For example, most medications target specific receptor systems that participate in complex signaling networks and are multifunctional. As a result, most medications have non-specific mechanisms of action that can lead to undesirable side effects and reduced efficacy. For example, leading antidepressants (i.e., the SSRIs) are plagued by significant sexual (decreased libido and delayed ejaculation), GI (nausea) and central nervous system (headache) side effects in at least 10% of the treatment population, and often require 4-6 weeks before onset of action. In addition, 30-40% of patients with depression do not respond to treatment with oral antidepressants. Thus, the identification of agents that specifically target neurogenesis provides opportunities for the development of more specific and efficacious treatments for depression by avoiding the receptors and pathways associated with the side effects of current antidepressants.

Neurogenesis-based treatments may also be effective in treating the cognitive decline associated with irradiation or chemotherapy treatments of a primary or metastatic brain tumor. Such declines occur in ∼50% of patients, but there are currently few successful treatments or preventive strategies. In animal models, radiation-induced brain injury is thought to be caused by hippocampal dysfunction resulting from decreased neurogenesis (Monje et al., 2002). Radiation induces a defect in the proliferative capacity of the neural progenitor cell population, while the remaining neural precursors adopt a non-neuronal glial fate. When grafted stem/precursor cells are implanted into radiated animal hippocampus there is a marked reduction in the differentiation of these cells into neurons, indicating that the microenvironment impacts neurogenesis. Radiation results in a marked increase in the number of activated microglia which secrete cytokines that influence neural precursor cell proliferation and fate (Monje et al., 2002). For example, microglia secrete interleukin (IL)-6, which has been shown to decrease in vitro neurogenesis, cell survival, and accumulation of neurons, likely due to reduced neuronal differentiation (Monje et al., 2003). Thus, IL-6 is a potent regulator of hippocampal neurogenesis. The identification of additional modulators of neurogenesis, including agents capable of stimulating neurogenesis, could potentially reverse the degenerative or cognitive effects of radiation and chemotherapeutic treatments.

Thus, the identification of therapeutic agents capable of modulating neurogenesis may lead to effective treatments for a variety of neoplastic diseases and/or neurological disorders. Moreover, exposure to pharmacological or other agents, such as food additives or environmental toxins, could interfere with neurogenesis, resulting in adverse consequences for brain functioning, including impaired cognition and memory. Accordingly, there is great need for sensitive and effective methods for assaying agents that modulate neurogenesis.

Citation of the above documents is not intended as an admission that any of the foregoing is pertinent prior art. All statements as to the date or representation as to the contents of these documents is based on the information available to the applicant and does not constitute any admission as to the correctness of the dates or contents of these documents.

### BRIEF SUMMARY OF THE INVENTION

The disclosed invention provides *in vitro* methods for identifying compounds or conditions that modulate neurogenesis (or "neurogenesis modulating agents" as defined below). In some embodiments, the neurogenesis modulating agents identified using methods of the disclosed invention modulate neurogenesis *in vivo.* The methods include "trophic" assays, which detect or identify agents or conditions that increase neurogenesis. The methods also include "toxic" or "toxicity" assays, which detect or identify agents or conditions that inhibit or decrease neurogenesis via toxicity to cells capable of neurogenesis.

Advantageously, methods of the disclosed invention provide tools with increased sensitivity, specificity and predictive value for detecting the effect of a wide range of treatment modalities on neurogenesis. In some embodiments, a method of the disclosed invention is used to develop improved antidepressants by identifying molecules or other treatments that modulate key steps in the neurogenesis cascade. The improved antidepressants include those that exhibit enhanced efficacy in the treatment of depression and related mood disorders relative to currently available medications.

Aspects of the disclosed invention include assays for neurosphere growth (or proliferation), which may be embodied in a quantifiable and/or high throughput method, and an assay based on neural cells in monolayer, or adherent, form as opposed to non-adherent neurospheres in suspension.

Thus in a first aspect, a method for identifying and characterizing neural stem cells in cell culture known as the neurosphere assay (NSA) is described herein. In the NSA, cells isolated from nervous tissues, such as the SVZ of the lateral ventricles or the DG of the hippocampus, proliferate in the presence of a mitogen, such as epidermal growth factor (EGF) and/or basic fibroblast growth factor (bFGF) as non-limiting examples, to form spherical clusters of cells termed neurospheres. Cultured neurospheres exhibit the primary characteristics of neural stem cells (NSCs), including the ability for self-renewal, or the ability to create progeny cells that retain the characteristics of the parental cells, and multipotentiality, or the ability to form more than one (up to all) of the cell types of the tissue from which the stem cell is derived. In the case of the central nervous system (CNS), multipotentiality includes the ability to form neurons and glial cells (astrocytes and oligodendrocytes). Multipotent NSCs may also have the ability to differentiate into other cell types, particularly endothelial cells, under some conditions.

In various embodiments, the disclosed invention provides improved methods for detecting agents that modulate neurogenesis in neurosphere cell culture. In some embodiments, culturing NSCs in neurospheres provides one or more advantages relative to methods using dissociated cells. For example, in some embodiments, neurospheres simulate certain conditions of the *in vivo* environment in which NSCs exist, such as cell to cell contacts with other NSCs, progenitor cells in various states of differentiation, and/or mature cells of the CNS. Accordingly, neurospheres may be used for detecting neurogenesis modulating agents and conditions that act through certain mechanisms, such as those involving cell to cell communication, including positive or negative feedback as non-limiting examples.

So in some embodiments, a NSA to detect growth, or proliferation, is described herein. In some cases, the method to detect neurosphere growth is based upon detection, or measurement, of enhanced survival of cells in a neurosphere. In other embodiments, the method is based upon detection, or measurement, of enhanced proliferation of cells in a neurosphere. Non-limiting examples of such methods disclosed herein include detecting or measuring 1) the size of a neurosphere; 2) the expression of cellular factors in a neurosphere, such as by ELISA, staining, or other assays as non-limiting examples; and 3) gene expression in a neurosphere.

The disclosed invention further includes methods for identifying an agent or condition that modulates neurogenesis by detecting or measuring neurosphere growth. Such methods comprise culturing a population of neurospheres comprising NSCs, such as human NSCs as a non-limiting example, and optionally isolating an individual neurosphere from the population of neurospheres. In addition to detecting or measuring neurosphere growth, the cultured neurospheres may be exposed to a test agent or condition followed by measuring at least one property of the neurosphere that is indicative of the nature and/or degree of neurogenesis. Properties that are indicative of neurogenesis include, as non-limiting examples, the expression of one or more genes; and the number and/or the proportion of neural stem cells, progenitor cells, or mitotic cells in one or more neurospheres or a test cell population, or sub-population, thereof. The measuring may be made in comparison to an identical population of cultured neurospheres, or an isolated neurosphere, that has not been exposed to the test agent or condition.

In additional embodiments, the neurosphere-based methods disclosed herein may include one or more features. Non-limiting examples of such features include dissociating the cells of, or in, a neurosphere; measuring at least one property of the dissociated cells, such as after exposure to a test agent or condition and neurosphere based measuring; and/or correlating a property of the dissociated cells with one or more properties of the neurosphere. In further embodiments, methods described herein include the additional features of: isolating a sub-population of neurospheres from the population of neurospheres; dissociating the cells in the sub-population of neurospheres; and determining the proportion of the dissociated cells that comprise NSCs. Additional embodiments include the step of comparing the proportion of NSCs in one or more isolated neurospheres exposed to the test agent or condition with the proportion of NSCs in a sub-population of neurospheres not exposed to the test agent or condition.

In a second aspect, methods are described herein for detecting agents and conditions that modulate neurogenesis involving the steps of: exposing a population of human neural stem cells in monolayer culture to a test agent or condition; and measuring at least one property of the neural cells that is indicative of the degree and/or nature of neurogenesis. In various embodiments, monolayer-based methods allow for the direct detection of neurogenesis modulating effect(s) on NSCs, for example via the identification, isolation, and/or enrichment of NSCs in the monolayer culture, and/or by controlling the microenvironment of the NSCs in the test cell population. In further embodiments, monolayer-based methods of the disclosed invention are used in conjunction with neurosphere-based methods to facilitate the detection and identification of neurogenesis modulating agents.

In some embodiments, the monolayer of NSCs used in a method disclosed herein has been passaged from a previous monolayer of NSCs. Thus the use of a monolayer of progeny cells derived from a previous monolayer is described herein. In other embodiments, the NSC monolayer is prepared from one or more neurospheres as described herein. Such a monolayer thus has not been passaged from a previous NSC monolayer.

Neural cells used in the practice of the disclosed invention, including both neurosphere and monolayer forms, are preferably isolated from a mammal, such as a mouse, rat, rabbit, or primate, and are most preferably isolated from human tissue. Because human NSCs have been reported to be difficult to proliferate and maintain in monolayer cell culture, in some embodiments, human neural cells are first cultured and serially passaged as neurospheres, which facilitates the isolation and expansion of neural stem cells in cell culture. Aspects of the disclosed invention involving monolayers of cells are based in part upon dissociating neurospheres followed by culturing them on an adherent surface, so as to produce a substantially uniform monolayer of cells that can be passaged as a monolayer suitable for detecting subsequent neurogenesis.

Therefore, and in some embodiments, methods of the disclosed invention comprise (a) culturing NSCs on a substrate as a monolayer; (b) contacting the cells with one or more test agents; (c) measuring at least one characteristic of the NSCs that is indicative of the nature and/or degree of neurogenesis; and (d) comparing the at least one characteristic of the NSCs to that of control NSCs that have been cultured in parallel to the test cells but have not been administered the test agent. An additional method disclosed herein comprises (a) contacting a monolayer of NSCs with one or more test agents; and (b) measuring at least one characteristic of the NSCs that is indicative of the nature and/or degree of neurogenesis. In some cases, these embodiments may be practiced with a monolayer of cells that has been passaged from a previous monolayer of cells. In other cases, the monolayer of cells may be one prepared from neurospheres.

In other embodiments, methods of the disclosed invention comprise (a) culturing NSCs as neurospheres; (b) dissociating the neurospheres and culturing the cells on a substrate as a monolayer; (c) contacting the cells with one or more test agents; and (d) measuring at least one characteristic of the NSCs that is indicative of the nature and/or degree of neurogenesis.

In further embodiments, a trophic or toxic assay method comprises dissociating NSCs from one or more neurospheres; plating them with deprivation of mitogens; and exposing them to a test agent or condition in the absence of mitogens to identify the agent or condition as being trophic or toxic to the cells. As described herein trophic compounds like histamine, and toxic agents like BAY-60-7550, have been identified.

In alternative embodiments, a method of the disclosed invention comprises the additional step of sorting the dissociated cells of a neurosphere to isolate NSCs, which are then cultured as a monolayer, as described below, treated with one or more test agents, and assessed for properties that are characteristic of neurogenesis. In some embodiments, sorting NSCs involves labeling NSCs or non-NSCs with cell type-specific labels, and sorting the cells using an automated process, such as fluorescent-activated cell sorting (FACS). In other embodiments, cell type-specific labeling provides a measure of the proportion of cells, for example in a neurosphere, that comprise NSCs or non-NSCs.

In additional embodiments, methods of the disclosed invention include culturing NSCs in the presence of one or more factors (hereinafter referred to as "constitutive factors") that facilitate the detection of neurogenesis modulating effects. The presence of one or more constitutive factors may advantageously facilitate the identification of a modulator of neurogenesis, such as by mimicking the *in vivo* milieu in which neurogenesis occurs. In some embodiments, constitutive factors useful in methods of the disclosed invention are molecules that are endogenous to regions of the brain where neurogenesis is known to occur or molecules that mimic and/or modulate the effects of such endogenous molecules. Regions where neurogenesis is known to occur include, but are not limited to, the dentate gyrus, the subventricular zone, and the olfactory bulb. Constitutive factors can comprise any type of molecule, treatment modality, or experimental condition. In some embodiments, constitutive factors comprise one or more neurotransmitters selected from the group including, but not limited to, serotonin, a serotonin precursor, norepinephrine, dopamine, AMPA, GABA, glutamate, and combinations of the above. Other molecules that may be a constitutive factor include, but are not limited to, mitogens, such as VEGF and IGF-1, and ions.

In a third aspect, methods are provided for identifying neural stem cells suitable for transplantation, wherein the methods include isolating a population of neural stem cells from a source of neural cells; exposing the neural stem cells to a test agent; and measuring the effect of the test agent on neurogenesis, wherein a significant effect indicates that neural stem cells from the source of the test population of cells are suitable or unsuitable for transplantation. In various embodiments, exposing neural stem cells to the test agent has a significant effect on the proportion of the neural stem cells that differentiate along a neuronal and/or a glial lineage; proportion of the neural stem cells that are mitotic cells; and/or the number of neural stem cells in the test population. For cells identified to be suitable for transplantation, additional cells from the source of neural cells may be transplanted to a subject. The transplanted cells may then undergo neurogenesis *in vivo* or optionally be induced to undergo neurogenesis by administration of one or more neurogenic agents or conditions known to the skilled person or as identified by the methods disclosed herein.

In additional embodiments, these methods are for identifying or generating populations and/or sources of neural stem cells suitable for transplantation *in vivo,* for example for therapeutic and/or experimental purposes. In various embodiments, such methods include the steps of: isolating a population of neural stem cells from a source of neural cells, such as a particular tissue, host, or cell line; exposing the neural stem cells to a test agent; and measuring one or more properties of the cells that are indicative of the suitability of the cells for transplantation. In various embodiments, properties indicative of suitability for transplantation include, but are not limited to, expression of one or more genes that are indicative of the degree and/or nature of neurogenesis, responsiveness or non-responsiveness to a test agent or condition, survivability in the presence of a test agent or condition, and propensity for differentiating into a particular lineage. The method may also include generating neural stem cells that have been identified using one of the recited methods, and transplanting those stem cells into an animal, such as a vertebrate as a non-limiting example. Additional examples include a mammal, such as a human.

Methods for the preparation of cells for transplantation are also disclosed. As a non-limiting example, and for neural cells observed to be capable of neurogenesis, additional cells from the source of neural cells may be induced to undergo neurogenesis ex *vivo* followed by transplantation to a subject. In some embodiments, the neural cells may have only been induced, while in other embodiments, the neural cells may have undergone neurogenesis as described herein prior to transplantation. Of course cells that have been induced, but not having undergone complete neurogenesis may also be transplanted. The induction of neurogenesis *ex vivo* may be by contact or exposure to one or more neurogenic agents or conditions known to the skilled person or as identified by the methods disclosed herein.

In a fourth aspect, methods are provided which yield improvements over existing methods. For example, to the extent that methods exist that include providing a population of cells that includes neural stem cells; contacting the population of cells with a test compound; and measuring at least one characteristic of the cells that is indicative of neurogenesis, methods described herein may provide an improvement to existing methods by contacting the population of cells with a neurotransmitter, such as a biogenic amine, in addition to the test agent.

In a fifth aspect, methods are provided for assaying a test compound for a potential neurogenic effect, wherein the methods include providing a population of cells *in vitro* that includes neural stem cells, wherein the cells are in contact with a growth medium; providing a neurotransmitter in the growth medium; contacting the population of cells with a test compound; and determining the effect of the test compound on the degree and/or nature of neurogenesis by the neural stem cells. In various embodiments, the neurotransmitter is a biogenic amine, or monoamine, such as dopamine, serotonin, or norepinephrine, or a compound that modulates the level or activity of one or more biogenic amines, such as a monoamine reuptake inhibitor, a monoamine receptor modulator, or a monoamine oxidase inhibitor. In alternative embodiments, a neurotransmitter that is not a biogenic amine may be.used.

In a sixth aspect, methods are provided for identifying one or more genes ("neurogenesis markers") expressed by cells in a test population, wherein the expression of the gene(s) indicates the modulation of neurogenesis by a test agent or condition. In some embodiments, methods for detecting neurogenesis markers include exposing a population of cells comprising neural stem cells to a test agent or condition; measuring at least one property of the cells that is indicative of the degree and/or nature of neurogenesis; measuring the expression of at least one gene by cells of the test population; and correlating gene expression with a measured property of the test cells that is indicative of neurogenesis.

In a seventh aspect, methods are provided for detecting a neuroprotective agent, wherein the methods include exposing a population of neural stem cells to an agent or condition that inhibits neurogenesis; exposing the neural stem cells to a test agent; and measuring the ability of the test agent to alleviate the inhibition of neurogenesis. In some embodiments, the exposure to an agent or condition that inhibits neurogenesis may be that which mimics an *in vivo* condition, such as one involving disease, to which an increase in neurogenesis is desirable as a therapeutic intervention. Thus non-limiting examples of such exposure include the presence of one or more opioids or inflammatory cytokines. Additional examples include cellular agents or factors that inhibit neurogenesis, such as angiotensin or angiotensin precursors released by reactive astrocytes. Alternatively, exposure to radiation or one or more toxic agents, such as a phosphodiesterase (PDE) inhibitor (like the PDE2 BAY-60-7550), may be used as an inhibitor of neurogenesis. Embodiments of these methods include those to identify an agent or condition, or combination thereof, which rescues or restores neurogenesis after the exposure to an anti-neurogenic agent or condition.

An eighth aspect of the disclosed invention includes methods for assessing whether a patient is responsive to treatment with a neurogenesis modulating agent, wherein the methods include obtaining a cell sample from a patient in need of treatment, wherein the sample comprises neural stem cells; exposing the sample to the neurogenesis modulating agent; and measuring the expression of a neurogenesis marker gene, wherein the expression or non-expression of the marker gene is predictive of whether the patient will be responsive to treatment with the neurogenesis modulating agent

Embodiments of the disclosed invention include automated, high throughput methods for measuring the effect of test agents and conditions on one or more properties of neural stem cells as a function of time. Advantageously, methods described herein provide an enhanced ability to detect certain neurogenesis modulating effects relative to known methods.

The details of additional embodiments are set forth in the accompanying drawings and the description below. Other features, objects, and advantages of the embodiments will be apparent from the drawings and detailed description, and from the claims.

### BRIEF DESCRIPTION OF THE DRAWINGS

**Fig. 1A** shows the well assignments for a 96-well plate for a typical experiment for measuring the effect of one or more test agents on the proliferation of NSCs in culture.
**Fig. 1B** shows a typical dose-response curve for a compound (naltrexone) that does not cause toxicity or proliferation when assayed under control conditions. The compound is thus neither toxic nor trophic for cells across a range of concentrations.
**Fig. 2A** is a dose-response curve showing the effect of varying concentrations of dopamine (squares) on the differentiation of cultured human neural stem cells (hNSCs) along a neuronal lineage. Background media values are subtracted and data is normalized with respect to a neuronal positive control. Dopamine exerted a minor effect on neuronal differentiation at concentrations of 1 µM or greater.

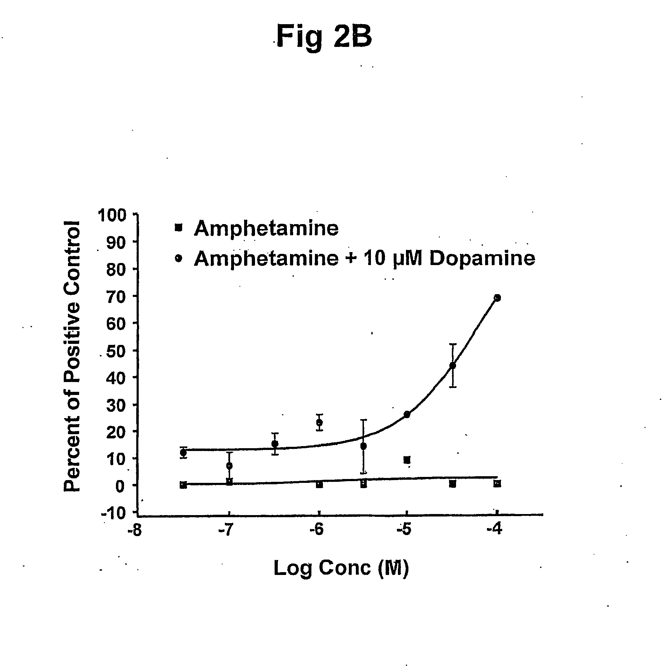

**Fig. 2B** is a dose-response curve showing the effect of varying concentrations of a test agent (amphetamine) on the differentiation of cultured human neural stem cells (hNSCs) along a neuronal lineage, in both the presence and absence of a constitutive factor. Background media values are subtracted and data is normalized with respect to a neuronal positive control. Amphetamine by itself (squares) did not have a significant effect on neuronal differentiation within the range of concentrations tested (up to 100 µM), whereas the combination of amphetamine and 10 µM dopamine as a "constitutive factor" (circles) significantly enhanced neuronal differentiation (EC50 of approximately 30 µM).
**Fig. 2C** is a dose-response curve showing the effect of varying concentrations of a test agent (methylphenidate) on the differentiation of cultured human neural stem cells (hNSCs) along a neuronal lineage, in both the presence and absence of a constitutive factor.
   Background media values are subtracted and data is normalized with respect to a neuronal positive control. Methylphenidate by itself (squares) had only a slight effect on neuronal differentiation at concentrations greater than about 1 µM, whereas the combination of methylphenidate and 10 µM dopamine as a "constitutive factor" (circles) significantly enhanced the degree of neuronal differentiation throughout the range of methylphenidate concentrations tested (from about 3 nM to about 10 µM).

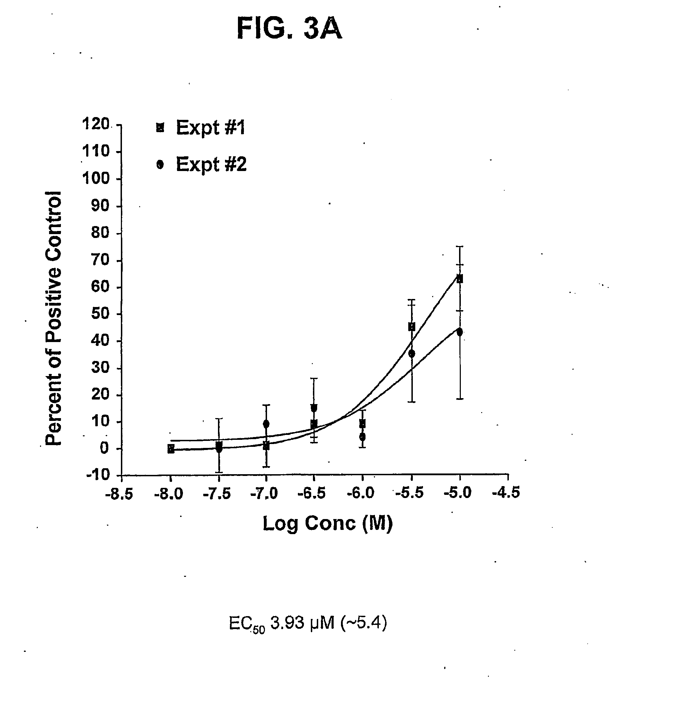

**Fig. 3A** is a dose-response curve showing the effect of varying concentrations of the neurotransmitter norepinephrine on the differentiation of cultured human neural stem cells (hNSCs) along a neuronal lineage. Background media values are subtracted and data is normalized with respect to a neuronal positive control. Data is shown for two independent experiments, experiment #1 (squares) and experiment #2 (circles). Norepinephrine significantly enhanced neuronal differentiation at micromolar concentrations, with a mean EC₅₀ of about 4 µM.

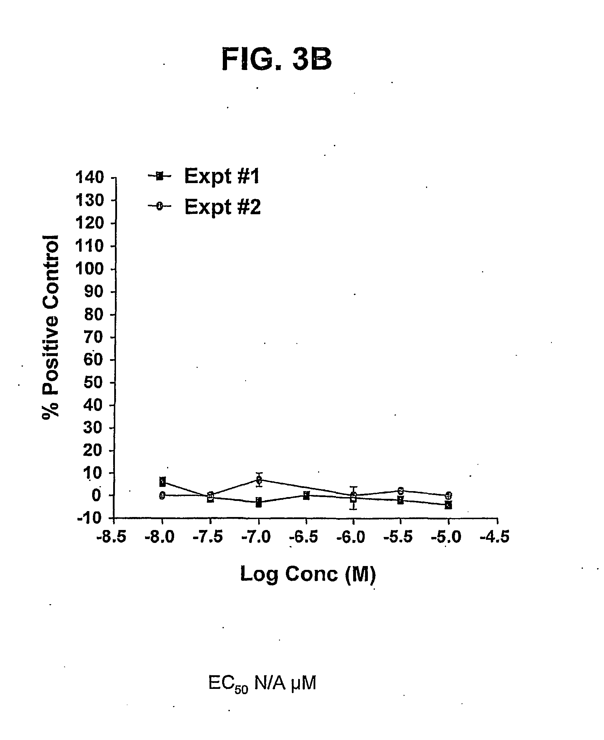

**Fig. 3B** is a dose-response curve showing the effect of varying concentrations of the neurotransmitter norepinephrine on the differentiation of cultured human neural stem cells (hNSCs) along an astrocyte lineage. Background media values are subtracted and data is normalized with respect to an astrocyte positive control. Data is shown for two independent experiments, experiment #1 (squares) and experiment #2 (circles). Norepinephrine had no effect on astrocyte differentiation within the range of concentrations tested (from about 0.01. µM to about 10 µM).

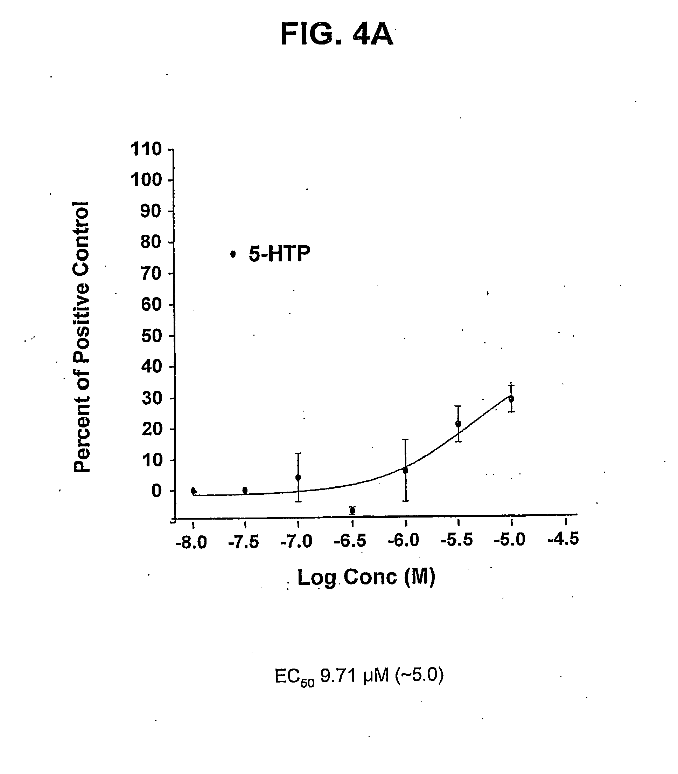

**Fig. 4A** a dose-response curve showing the effect of varying concentrations of the serotonin precursor 5-Hydroxy L tryptophan (5-HTP) (circles) on the differentiation of cultured human neural stem cells (hNSCs) along a neuronal lineage. Background media values are subtracted and data is normalized with respect to a neuronal positive control. 5-HTP significantly enhanced neuronal differentiation at concentrations of about 4 µM or greater, while having no significant effect at lower concentrations.

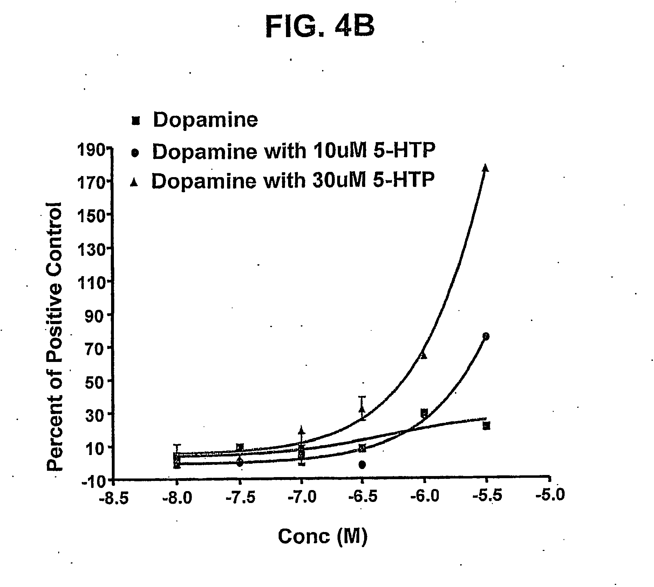

**Fig. 4B** is a dose-response curve showing the effect of varying concentrations of a test agent (the neurotransmitter dopamine) on the differentiation of cultured human neural stem cells (hNSCs) along a neuronal lineage, in both the presence and absence of a constitutive factor. Background media values are subtracted and data is normalized with respect to a neuronal positive control. Dopamine by itself (squares) had only a slight effect on neuronal differentiation within the range of concentrations tested (from about 0.01 µM to 10 µM), whereas the combination of dopamine and either 10 µM (circles) or 30 µM (triangles) 5-HTP as a "constitutive factor" significantly enhanced neuronal differentiation, with EC₅₀ values of from about 3 nM to about 10 µM.

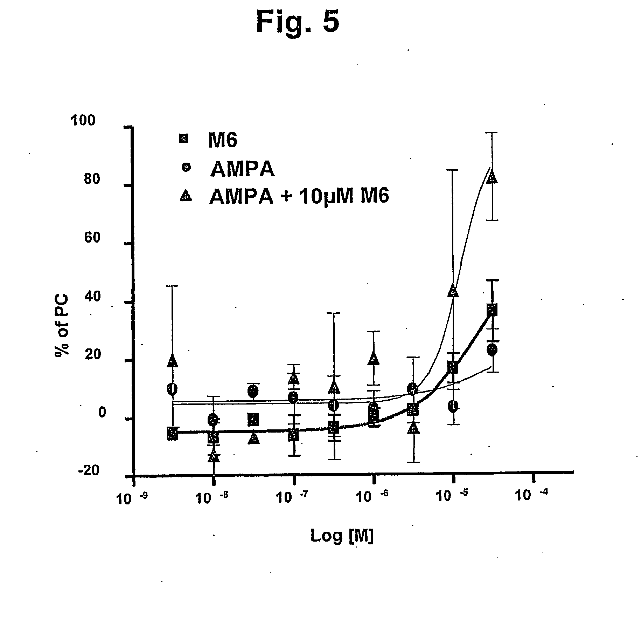

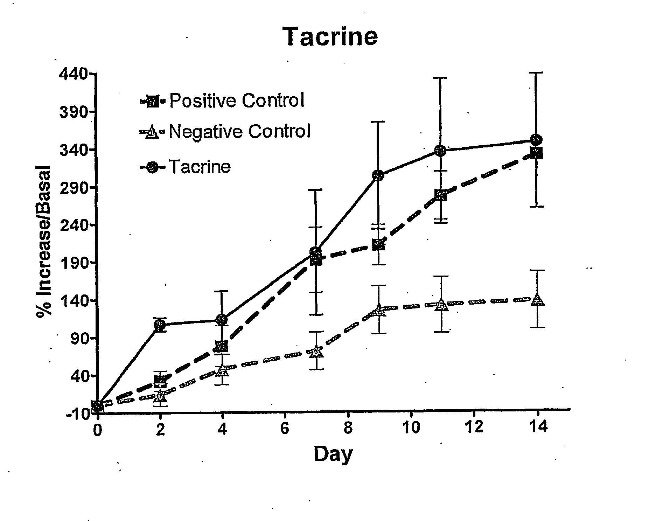

**Fig. 5** is a dose-response curve showing the effect of varying concentrations of AMPA on the differentiation of cultured human neural stem cells (hNSCs) along a neuronal lineage, in both the presence and absence of a second factor. Background media values are subtracted and data is normalized with respect to a neuronal positive control. AMPA by itself (squares) had only a slight effect on neuronal differentiation at the highest concentration tested (about 30 µM), whereas the combination of AMPA and 10 µM of the nootropic compound M6, or cyclo-(Pro-Gly), (circles) led to significantly enhanced levels of neuronal differentiation throughout the range of AMPA concentrations tested (from about 3 nM to about 10 µM).
**Fig. 6** shows that tacrine promoted NSC growth in neurospheres.

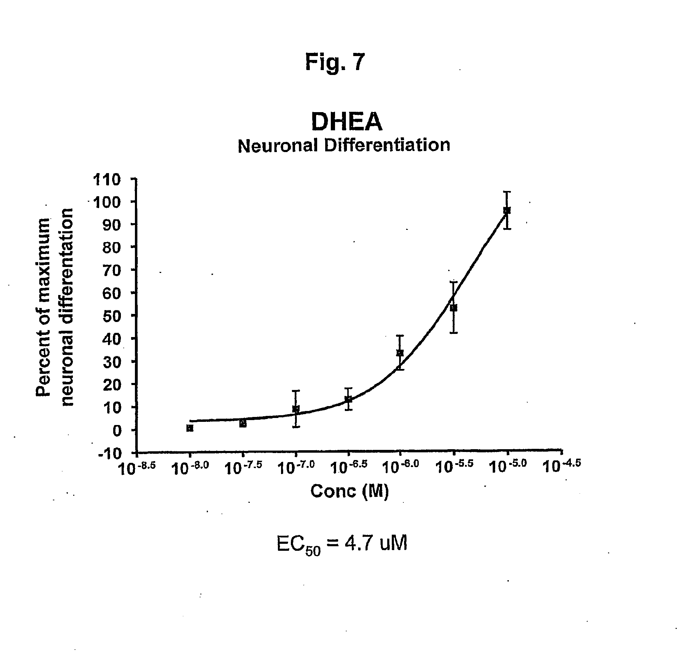

**Fig. 7** shows that DHEA promotes differentiation of human NSCs cultured as monolayers in 96-well plates.

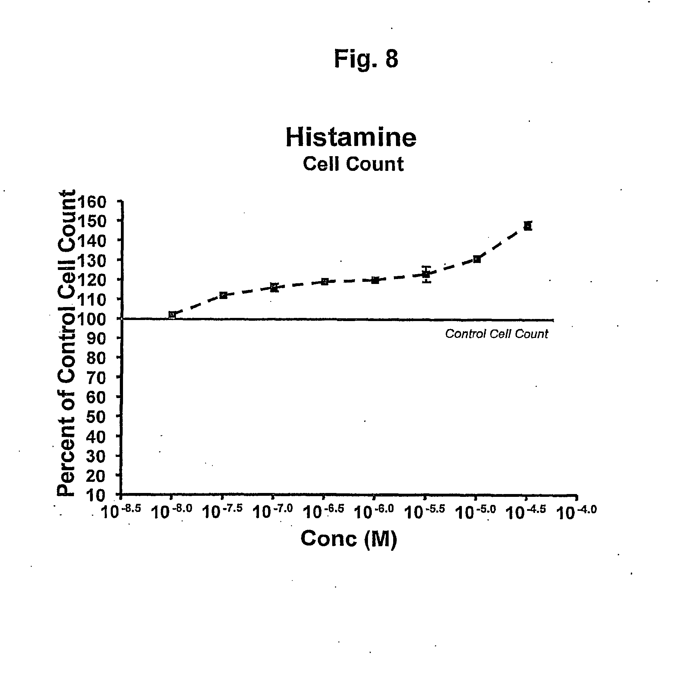

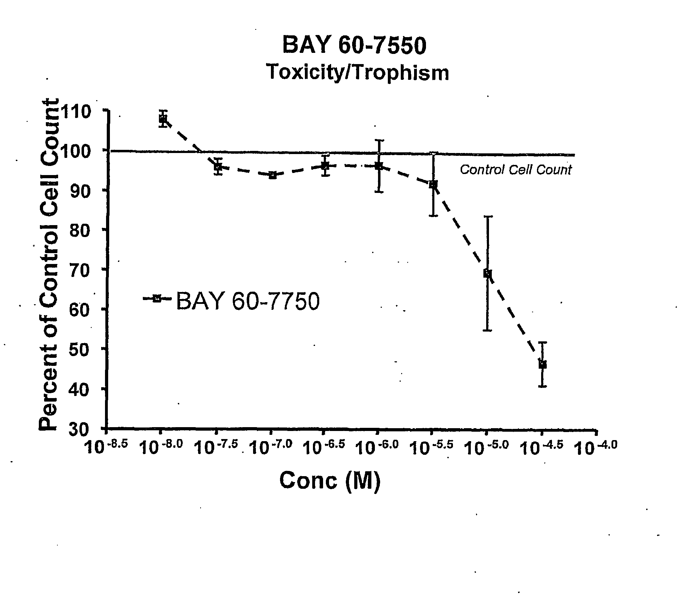

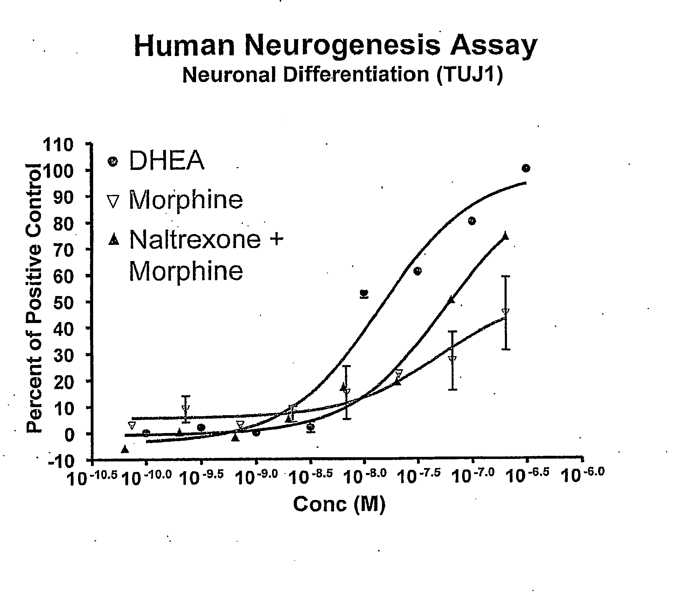

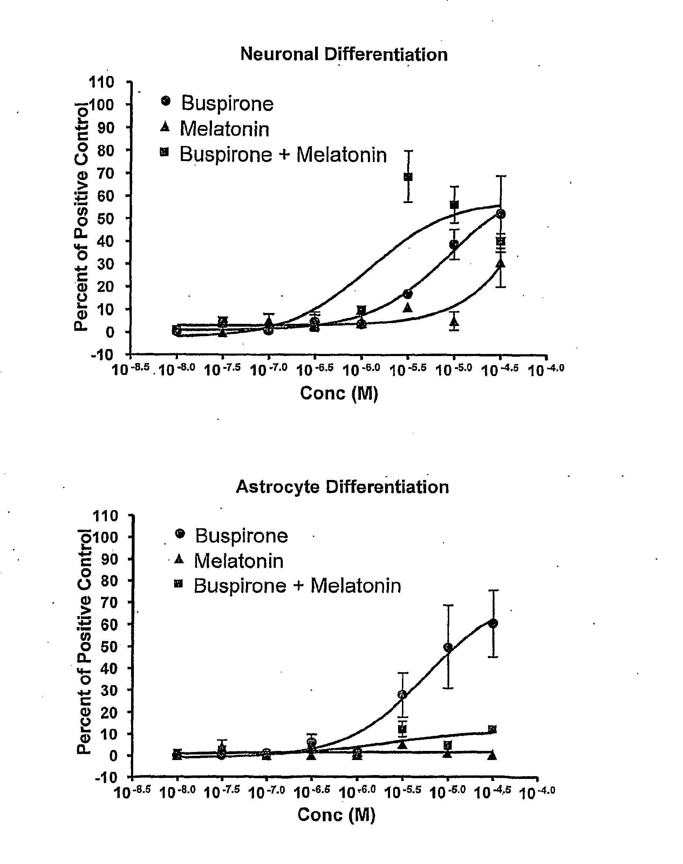

**Fig. 8** shows that histamine promotes increased cell proliferation of human NSCs cultured as monolayers in 96-well plates. Thus histamine is a trophic compound.
**Fig. 9** shows that the PDE2 inhibitor BAY-60-7550 induces toxicity in human NSCs cultured as monolayers in 96-well plates. Thus this inhibitor is a toxic compound.
**Fig. 10** shows that naltrexone rescues opioid-induced inhibition of NSC neuronal differentiation (the filled circles represent data from cells treated with DHEA as a positive control; the filled triangles represent data from cells treated with both naltrexone and morphine; the open triangles represent data from cells treated with morphine alone). The presence of naltrexone with morphine restores cell differentiation to a level closer to that of the positive control.
**Fig. 11** shows that a combination of buspirone and melatonin results in neuronal differentiation (upper panel) while inhibiting differentiation into astrocytes (lower panel).

### DEFINITION OF CERTAIN TERMS USED IN THE DESCRIPTION

"Neurogenesis" is defined herein as proliferation (cell growth), differentiation, migration and/or survival of a neural cell *in vivo* or *in vitro.* Embodiments of the disclosed invention include the detection or measurement of either proliferation or differentiation or survival as non-limiting indicators of neurogenesis. Neurogenesis is intended to cover neurogenesis as it occurs during normal development, as well as neural regeneration that occurs following disease, damage or therapeutic intervention.

Neurogenesis is distinct from "astrogenesis," which refers to the proliferation, differentiation, migration and/or survival of an astrocytic cell *in vivo* or *in vitro.* Non-limiting examples of astrocytic cells include astrocytes, activated microglial cells, astrocyte precursors and potentiated cells, and astrocyte progenitor and derived cells. An astrocyte may be an adult, fetal, or embryonic astrocyte and may be located in the central nervous system or elsewhere in an animal or human being, including a tissue such as neural tissue. Astrogenesis includes the proliferation and/or differentiation of astrocytes as it occurs during normal development, as well as astrogenesis that occurs following disease, damage or therapeutic intervention, such as by treatment with high doses of an astrogenic agent like buspirone as described herein.

Neurogenesis optionally includes the generation of oligodendrocytes which refers to the proliferation, differentiation, migration and/or survival of an oligodendrocytic cell *in vivo* or *in vitro.* Non-limiting examples of oligodendrocytic cells include oligodendrocytes, , oligodendrocyte precursors and potentiated cells, and oligodendrocyte progenitor and derived cells. An oligodendrocyte may be an adult, fetal, or embryonic oligodendrocyte and may be located in the central nervous system or elsewhere in an animal or human being, including a tissue such as neural tissue. Generation of oligodendrocytes includes the proliferation and/or differentiation of oligodendrocytes as it occurs during normal development, as well as the generation or protection of oligodendrocytes that occurs following disease, damage or therapeutic intervention.

The proliferation, or growth, of cells as described herein refers to the ability of a population of one or more cells to replicate and increase their number(s) via mitosis. This may be measured by the counting of cell numbers or an increase in the overall cell mass such as in the case of an increase in neurosphere size. An agent, compound, or condition that decreases or inhibits cell growth is "toxic" as used herein. Forms of toxicity include both inhibition of mitosis, such as by a cytostatic effect, and lethality, such as by a cytotoxic effect. An agent, compound, or condition that increases the growth of cells may be termed a "trophic" agent. A method based on the detection or measurement of a decrease or inhibition of cell growth may be termed a "toxicity assay" while a method based on detecting or measuring an increase in cell growth may be termed a "tropism" or "proliferation" or "growth" assay.

The term "neural cell" includes neural stem cells (NSCs), neural progenitor cells, and progeny of such stem and progenitor cells, including differentiated cells that originate therefrom. In one embodiment, the neural cell is an adult, fetal, or embryonic neural stem cell or population of cells. In some embodiments, the neural cell is an adult, fetal, or embryonic progenitor cell or population of cells, or a population of cells comprising a mixture of stem cells and progenitor cells. Neural cells include all brain stem cells, all brain progenitor cells, and all brain precursor cells.

A "neurogenesis modulating agent" is defined as an agent or reagent that can promote, inhibit, or otherwise modulate the degree or nature of neurogenesis *in vivo* or *ex vivo* relative to the degree or nature of neurogenesis in the absence of the agent or reagent.

Modulation of neurogenesis refers to the increase or decrease of neurogenesis in a cell or population of cells capable of neurogenesis. Non-limiting examples of modulation include a direct increase or decrease in neurogenesis, such as among a population of neural cells, or an increase or decrease in an inhibitor of neurogenesis. A representative, and non-limiting, example of the latter is the decrease in astrocytes or astrogenesis.

The term "stem cell" (e.g., neural stem cell (NSC)), as used herein, refers to an undifferentiated cell that is capable of self-renewal and differentiation into neurons, astrocytes, and/or oligodendrocytes.

The term "progenitor cell" (e.g., neural progenitor cell), as used herein, refers to a cell derived from a stem cell that is not itself a stem cell. Some progenitor cells can produce progeny that are capable of differentiating into more than one cell type.

### DETAILED DESCRIPTION OF MODES OF PRACTICING THE INVENTION

Neural stem and/or progenitor cells suitable for use in the assay methods described herein can be obtained from mammals, including humans (post-mortem or following surgery) and experimental animals (such as rodents, non-human primates, dogs, cats and the lilce). Neural stem and/or progenitor cells can also be obtained from other vertebrates, including reptiles, amphibians, fish and birds, or from invertebrates. The human or animal can be male or female, can be fetal, young, adult or old, and can be normal or exhibiting or susceptible to a neural disease or disorder. Thus, in some embodiments, neural cells used in methods of the disclosed invention are isolated from a test animal that has been administered one or more test agents. In other embodiments, neural cells are isolated from a subject diagnosed with a neurological condition. In some aspects, the neurological condition is a condition associated with a change in the nature and/or degree of neurogenesis, or is a neurodegenerative disease.

Human NSCs can be expanded in culture over long periods of time to generate stable cell lines of multipotent NSCs. Human NSCs from such cell lines can be preserved by freezing, and subsequently re-constituted for experimental use. Thus, in some embodiments, NSCs from an established cell line are used in the practice of the disclosed invention. The isolation and purification of human and rodent NSCs is describe in U.S. Patent Nos. 6,767,738, 6,265,175, 6,013,521 and 5,766,948, which are herein incorporated by reference in their entirety.

NSCs can be obtained from any neural tissue that contains neural stem or progenitor cells. Exemplary tissues include the olfactory bulb (OB), the dentate gyrus (DG) of the hippocampus, and the subventricular zone (SVZ) of the lateral ventricles. Methods for obtaining NSCs from such tissues are known to the skilled person (see, for example, U.S. Pat. No. 5,753,506; and Gritti et al., J. Neurosci. 16:1091-1100 (1996)). Cells isolated from dissected tissues can be identified as NSCs by culturing the cells as neurospheres (see e.g., Example 1) and demonstrating that the cells-have one or more properties characteristic of NSCs. In some embodiments, the cells of, or in, the neurospheres are passaged to demonstrate their ability to form secondary, tertiary, or additional generations of neurospheres (i.e., the ability to self-renew). In additional aspects, the cells of, or in, the neurospheres are cultured under conditions such that they differentiate into neurons, astrocytes, and/or oligodendrocytes.

In addition to primary cells, neural stem and/or progenitor cell lines can be used in the disclosed neurogenesis assays and methods. Non-limiting examples include MHP36 cells of mouse hippocampal origin (Gray et al., Philos. Trans. Royal Soc. Lond. B. Biol. Sci. 354:1407-1421 (1999)), CSM14.1 cells of rat mesencephalic origin (Haas et al., J. Anat. 201:61-69 (2002)), and embryonic stem cells differentiated along the neural lineage.

As described herein, one aspect of the disclosure is the use of neurospheres to identify and/or characterize NSCs, such as human NSCs. These methods may also be referred to as neurosphere assays or NSAs. Methods for culturing rodent and human NSCs as neurospheres are known to the skilled person. A typical protocol is described in Example 1. In the illustrated, non-limiting embodiment, isolated neural cells are cultured in the presence of a mitogen, such as epidermal growth factor (EGF) and/or basic fibroblast growth factor (bFGF), whereupon they divide and form spherical clusters of cells referred to as neurospheres. In some embodiments, neurospheres comprise a mixture of cell types at various stages of differentiation, including NSCs, progenitor cells, and differentiated neurons and glial cells. Thus, in various disclosed embodiments, neurospheres used in methods provided herein are serially passaged, for exampled by dissociating the constituent cells and culturing them in the presence of one or mitogens. Useful mitogens include, but are not limited to, EGF, bFGF, FGF, VEGF, and LIF.

Neurospheres can be dissociated physically, for example by chopping, or enzymatically, for example with trypsin. Advantageously, differentiated and differentiating cells die upon re-plating of the dissociated cells, so that successive generations of neurospheres (secondary, tertiary, etc.) comprise increasing proportions of NSCs. In various embodiments, neurospheres used in methods provided herein are passaged at least two or three times, or more. In other embodiments, they are passaged at least four or five times, or even more, such as at least six or more times to ensure that neurospheres used in methods described herein are comprised substantially of multi-potent NSCs, as opposed to progenitor cells having sphere-forming potential.

Example 2 describes an automated, high-throughput method for detecting the effect of a test agent or agents on one or more aspects of cultured neurospheres. Significantly, the technique allows for the observation, detection, and measurement of various aspects of individual neurospheres under controlled conditions as function of time. For example, in the embodiment described in Example 2, a single neurosphere is observed over time in each well of a 96-well plate under non-differentiating conditions, and the proliferation of the neurospheres is detected by measuring their size (e.g., as indicated by their diameter or other dimension(s)) as a function of time.

The disclosed invention includes a method for measuring the growth, or proliferation, of NSCs by the use of neurospheres of a specified, or limited, size range, such as that determined by inspection of a neurosphere's visible cross-sectional area. In some embodiments, the methods comprise the use of neurospheres have an area of less than about 1.4 mm² such as neurospheres with an area of at least about 0.01 mm² to about 1.4 mm². In other embodiments, neurospheres with an area of about 0.01, about 0.02, about 0.04, about 0.05, about 0.06, about 0.08, about 0.1, about 0.2, about 0.3, about 0.4, about 0.5, about 0.6, about 0.7, about 0.8, about 0.9, about 1.0, about 1.1, about 1.2, about 1.3, or about 1.4, mm² may be used in the methods. Of course neurospheres with a size range delimited by any of the above values, such as from about 0.1 to about 0.2 mm², about 0.1 to about 0.3 mm², about 0.1 to about 0.4 mm², about 0.1 to about 0.5 mm², about 0.1 to about 0.6 mm², about 0.1 to about 0.7 mm², about 0.1 to about 0.8 mm², about 0.1 to about 0.9 mm², about 0.1 to about 1.0 mm², about 0.2 to about 0.3 mm², about 0.2 to about 0.4 mm², about 0.2 to about 0.5 mm², about 0.2 to about 0.6 mm², about 0.2 to about 0.7 mm², about 0.2 to about 0.8 mm², about 0.2 to about 0.9 mm², about 0.2 to about 1.0 mm², about 0.3 to about 0.4 mm², about 0.3 to about 0.5 mm², about 0.3 to about 0.6 mm², about 0.3 to about 0.7 mm², about 0.3 to about 0.8 mm², about 0.3 to about 0.9 mm², about 0.3 to about 1.0 mm², about 0.4 to about 0.5 mm², about 0.4 to about 0.6 mm², about 0.4 to about 0.7 mm², about 0.4 to about 0.8 mm², about 0.4 to about 0.9 mm², about 0.4 to about 1.0 mm², about 0.5 to about 0.6 mm², about 0.5 to about 0.7 mm², about 0.5 to about 0.8 mm², about 0.5 to about 0.9 mm², about 0.5 to about 1.0 mm², about 0.6 to about 0.7 mm², about 0.6 to about 0.8 mm², about 0.6 to about 0.9 mm², and about 0.6 to about 1.0 mm² may be used in the practice of the disclosed methods. Methods for the dissociation, or sizing, of neurospheres into those of these specified sizes, or size ranges, are known to the skilled person and disclosed herein. Non-limiting examples include the use of manual dissociation using a tissue chopper.

Neurospheres of such sizes may be advantageously used in automated or partially automated methods, such as by the depositing of one or more neurospheres with the above size range in a well of a multi-well plate. Non-limiting examples of plates include those with 384 or 1536 wells. The depositing of neurospheres may be by any convenient means, including the use of the conditioned media in which the neurospheres were grown prior to, or during, their dissociation. Of course the neurospheres may be optionally fed by the addition of fresh medium (like maintenance medium as a non-limiting example) after dispensing into an individual well. In some embodiments, the fresh medium may be from about 50% to about 75% of the volume in the well. The neurospheres may then be exposed to one or more test agents (or compounds) via the medium and/or one or more test conditions.

The effect, or lack thereof, of a test agent or test condition of the neurospheres is then assayed by measuring the size of the neurosphere(s) of a well over time. In some embodiments, the measuring is for the increase in area on a daily, every two days, every three days, or less frequent basis for periods of several days to about 1, about 2, or about 3 or more weeks. Of course an increase in neurosphere size is indicative of growth while a lack of increase or a decrease in size is indicative of no growth and/or cell death. In some embodiments, the measuring is of the entire content, or field, of a well such that the one or more neurospheres therein are all visible and measured. This advantageously allows the effect of a test agent or test condition on all the neurospheres of a well to be measured.

Optionally, the capturing of the entire image of a well as a visual field may be automated via the use of low magnification and the use of a cell analyzer and plate reader under brightfield light conditions. Non-limiting examples of low magnification include from about 2x, about 3x, about 4x, or about 5x up to 10x. The automation may include the measurement of neurosphere size, such as by measuring neurosphere diameter as a non-limiting example, in each well under analysis.

The disclosed invention thus includes a method for identifying an agent or condition that modulates neurogenesis comprising exposing a neurosphere having a cross-sectional area of less than about 0.6 mm², such as from at least about 0.2 mm² to about 0.6 mm², to a test agent or test condition; and identifying said test agent or test condition as modulating neurogenesis in said neurosphere after measuring a property indicative of neurogenesis in said cells. The modulating may produce an increase, a decrease, or a lack of change in the size of the neurosphere over time. Thus a property of the isolated neurosphere comprises one or more dimensions of the neurosphere.

In some embodiments, the measurement of neurosphere size may be automated in whole or in part, such as by the use of an apparatus to visualize the neurosphere as described herein and/or the use of an apparatus to measure neurosphere size. In other embodiments, the neurospheres may comprise human neural stem cells while in further embodiments, the measuring is carried out at one or more, such as two or more, time points after exposure to the test agent or condition.

In other embodiments, proliferation can be measured using methods described herein, or other techniques known to the skilled person. In some embodiments, individual neurospheres are isolated from a population of neurospheres, and the isolated neurospheres are treated with one or more test agents or conditions. In some embodiments, neurospheres are "isolated" where they are maintained under conditions that allow for observation of the same neurosphere over time.

In various embodiments disclosed herein, neurosphere-based methods have the ability to detect effects on neurogenesis not previously known to be detectable, such as time-dependent effects (e.g., transient changes) or effects that occur via multi-stage processes (e.g., in response to a signaling cascade). For example, in some embodiments, neurospheres are treated with compound or treatment modality A and observed over a defined period, followed by treatment with compound or treatment modality B (or a mixture of A and B). In additional embodiments, the cells comprising the neurospheres are subsequently dissociated (typically after measuring one or more aspects of the neurospheres), and one or more characteristics of the cells measured.

For example, the neurospheres can be dissociated and plated in monolayer culture (e.g., as described in Example 3), and the degree and/or nature of their differentiation measured using the techniques described herein, or other techniques known to the skilled person. In further embodiments, the composition of cell-types comprising the neurospheres under non-differentiating conditions are determined, for example using cell type-specific labeling as described below, or another technique known to the skilled person. Thus, the neurosphere-based methods of the disclosed invention allow for the measurement of the effect of treatment modalities on individual neurospheres, as well as the subsequent evaluation of these effects in light of one or more characteristics of the cells comprising such neurospheres (e.g., cell-type composition, developmental fate, etc.). Advantageously, methods of the disclosed invention have a substantially enhanced sensitivity in the detection of changes in the degree/and or nature of neurogenesis relative to prior art methods. Moreover, the methods of the disclosed invention offer automated, high throughput methods for quickly and economically screening the effects of a range of treatment modalities on neurogenesis.

Another aspect of the disclosure is a method for detecting neurogenesis modulating agents and/or conditions by measuring the effect of a test agent or condition on one or more aspects of NSCs in monolayer culture. In various embodiments, the arrangement of the cells in a monolayer, as opposed to the spherical clusters of cells comprising neurospheres, enhances the ability to detect certain neurogenesis modulating agents and/or conditions relative to methods conducted in a multicellular environment. Without being bound by a particular theory, it is believed that neurospheres often comprise a mixture of neural stem cells, progenitor cells, and/or differentiated cells, and that this heterogeneous composition can make it difficult to interpret experimental results. For example, under certain conditions, the effect of a test agent on one or more properties of a neurosphere may be mediated by non-NSCs (e.g., due to neighboring cells exerting effects on NSCs via cell-cell contacts, secreted factors, etc.). Moreover, even serially passaged neurospheres can comprise a substantial proportion of non-NSCs, for example because NSCs undergo spontaneous differentiation and/or asymmetric cell division (e.g., giving rise to one NSC and one progenitor cell) upon passaging. Advantageously, monolayer-based methods described herein facilitate detection of certain neurogenic effects, for example by allowing greater control over the microenvironment of neural stem cells in the test population and/or allowing the effects of test agents on NSCs to be directly observed. Thus, in some embodiments, effects observed in monolayer-based methods are substantially attributable to an effect of the test agent or condition on NSCs, without substantial contribution by neighboring cells or other microenvironmental variables.

This aspect of the disclosure is based in part on a method for the stable culturing of neural cells, such as human neural stem cells, as a monolayer. Exemplary protocols for culturing human NSCs in monolayer culture is described in Example 3. In some embodiments, NSCs are isolated from neurospheres by enzymatic dissociation, such as with the enzymatic activity of ACCUTASE™, and trituration with a pipette. The cells are then washed, counted, and plated on surfaces coated with poly-lysine and laminin. In some embodiments, the poly-lysine comprises a greater proportion of poly-L-lysine than poly-D-lysine, and preferably comprises poly-L-lysine substantially free from poly-D-lysine. In other embodiments, the cells are isolated directly from neural tissues or are derived from an established NSC line. The long term culture and passaging of human NSCs in monolayer culture is accomplished in media containing EGF, bFGF, heparin and leukemia inhibiting factor (LIF). The proportions of these growth factors utilized in Example 3 have been optimized for the growth and maintenance of human NSCs. Cells are passaged by dissociating them from the substrate enzymatically, such as with ACCUTASE™, and re-plating the cells on poly-L-lysine and laminin-coated surfaces.

Importantly, the methods to culture NSCs as a monolayer allows the passaging of the cells such that the stem cell nature is preserved. This permits the exposure of the cells to specific factors in a defined medium, as well as culture on a defined substrate, to facilitate use of the cells in the detection or measurement of neurogenesis. The disclosure thus includes a method of culturing NSCs comprising passaging the cells as a monolayer culture on coated plates in the presence of the factors and media as described herein. The cultured, or passaged, cells may be used in the monolayer-based methods and assays as described herein. In some embodiments, the cells are used in a method for identifying an agent or condition that modulates neurogenesis. The method may comprise exposing a monolayer cell culture comprising human neural cells to a test agent or condition, and identifying the test agent or condition as modulating neurogenesis in said cells after measuring a property indicative of neurogenesis in said cells. In some embodiments, the neural cells comprise human neural stem cells (NSCs). Alternatively, cells isolated from neurospheres and converted into monolayer culture, without passaging as a monolayer culture, may be used in the practice of the disclosed methods.

In further embodiments, the cells to be plated as a monolayer are exposed to the agent or condition prior to their adherence to a solid surface. After attachment, the cells are then cultured as described herein. Where an agent is used, additional agent may be introduced to the cells on a subsequent day of culture and prior to assaying the cells for the effect of the agent. Non-limiting examples of the invention include a method that is conducted over the course of several days, such as about 7 days or more, where the last day is the measuring or detecting of a property indicative of neurogenesis. Methods for longer periods include about 9, about 11, about 13, about 15, about 17, about 19, or about 21 days or longer. Where an agent is used, the cells may be exposed to the agent on any subsequent day, such as day 1, day 2, day 3, day 4, day 5, or day 6 where the method is conducted for 7 days. Where a condition is used, the cells may be exposed and maintained under the condition for the duration of the method.

As described herein, methods of the disclosed invention may comprise the measuring of one or more characteristics of neural cells that are indicative of the degree and/or nature of neurogenesis, and comparing the measured characteristics to one or more control groups of cells. In various embodiments, the characteristic of the NSCs that is indicative of the nature and/or degree of neurogenesis comprises measuring the proliferation, differentiation, migration and/or survival of a neural cell *in vitro* and/or *in vivo.* The proliferation, differentiation, migration and/or survival of NSCs and/or progenitor cells can be measured using techniques described herein, and/or using other techniques known to the skilled person.

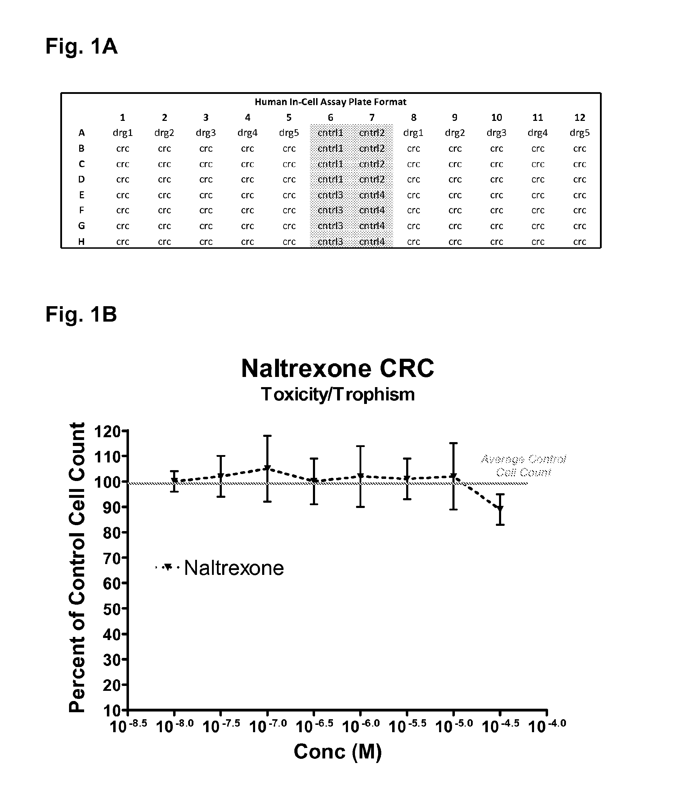

In some embodiments, the characteristic that is indicative of the nature and/or degree of neurogenesis is the proliferative capacity of NSCs. Example 4 describes one embodiment of an automated, high-throughput method for measuring the proliferation of NSCs in monolayer culture under a variety of conditions. In one embodiment, neurospheres are enzymatically dissociated with ACCUTASE™ as described above, counted, and cultured as monolayers on poly-L-lysine and laminin-coated multi-well plates. In a typical -experiment, 50,000 cells are plated per 100µl well. To measure the effect of a test agent on the proliferative capacity of the cells, one or more test agents are added and the cells are cultured in the presence of mitogens. In Example 4, five test agents are tested in duplicate in a 96-well plate to produce eight-point dose-response curves. After maintaining the cells in culture for a defined period, the cells are fixed, stained, and counted using an automated plate reader and customized software. Dose response curves in the presence of test agents are compared to controls in the absence of the test agent A typical dose-response curve under control conditions, or with an agent that is neither toxic nor trophic, is shown in Fig. 1B.

Proliferation can also be measured by the ability of cells to incorporate ³H thymidine, bromodeoxyurine (BrdU, a thymidine analog), or another indicator of proliferative activity. Cells can also be assessed for their expression of proliferation markers, such as proliferating cell nuclear antigen (PCNA) or cdc2. In these embodiments, gene expression can be measured using reporter systems described below.

The characteristic indicative of the nature and/or degree of neurogenesis may also comprise the ability of the NSCs to differentiate into neurons, astrocytes, oligodendrocytes, and/or another cell type, such as endothelial cells. Cultured NSCs generally differentiate when cultured in the absence of mitogens. Example 5 describes an automated, high-throughput method for measuring NSC differentiation similar to the proliferation assay of Example 4. In one embodiment, neurospheres are enzymatically dissociated, counted, and plated in monolayer culture as described for the proliferation assay, except that the cells are cultured in the absence of EGF and bFGF. After culture for a defined period, the cells are fixed and labeled, for example with antibodies specific for particular cell types. For example, glial fibrillary acidic protein (GFAP) antibodies specifically label astrocytes, β-tubulin III (TUJ-1) and neurofilament antibodies, such as NF-200, specifically label neurons, and the O1 and O4 antibodies specifically label oligodendrocytes. Other markers specific for cells of various lineages are known to the skilled person, and antibodies thereto are commercially available or can be generated by known methods. Unless the primary antibody is labeled, the cells are then generally contacted with labeled secondary antibodies, such as enzymatically or fluorescently labeled antibodies, and the cells are visualized or sorted. Methods to immuno-label cells, as well as methods to detect and sort immunolabeled cells, are well known to the skilled person. The effect of test agents on the differentiation of cells in monolayer culture can be measured as, for example, the proportion of cells plated that differentiate, or the proportion of cells that differentiate into one or more specific lineages relative to control cells.

A high-throughput, high content assay to evaluate the effects of different agents upon the differentiation of NSCs is also disclosed. The assay is based on the availability of stable monolayer NSC culturing methods as described herein. The assay may be optionally miniaturized as a differentiation detection system. Example 5 herein includes one embodiment of such a differentiation detection assay which permits multiple analyses in a concentration response curve format. The assay method may comprise plating a fixed density of NSCs, such as human NSCs, in the wells of a multi-well plate. In some embodiments of the disclosed invention, the specific density is about 60,000, about 70,000, about 80,000, or about 90,000 cells/cm². In other embodiments, a density of about 78,125 cells/cm² is used. Non-limiting examples of a multi-well plate for use in the method include 96-, 384-, and 1536-well plates coated with the substrate of 10 µg/ml poly-D-lysine and 50 µg/ml mouse Laminin.

The cells may be cultured in mitogen free test media or exposed to a test differentiating agent or condition as described herein immediately upon plating of cells. Stable, differentiation-compatible culture may be used with automated equipment known to the skilled person, such as equipment capable of replacing 50% of the media with newly prepared (fresh) media, optionally with a test differentiation agent or compound, between 3 to 4 days after plating. Non-limiting examples of conditions that may affect neurogenesis include oxygen concentration, pH, and carbon dioxide concentration that cells are exposed to. With respect to oxygen concentration, a concentration that better mimics the in *vivo* environment or brain milieu, such as a lower oxygen tension (to about 5 to 8% as a non-limiting example) may be used in the practice of the methods disclosed herein, whether monolayer or neurosphere based.

Measurement of the resulting NSC differentiation may be performed by fixation and staining as described herein and known to the skilled person. In some embodiments, use of automated equipment to take multiple pictures per well, at multiple wavelengths, may be used. Quantification of neuronal differentiation may be by measuring the amount of Tuj 1 staining and dividing the number by the number of cells as determined through automated counting of Hoechst stained cell nuclei. Quantification of astocytic differentiation may be by measuring the amount of GFAP staining and dividing the number by the number of cells as determined through automated counting of Hoechst stained cell nuclei. A concentration response curve demonstrating increased differentiation of NSCs into neurons with increased concentrations of serotonin (5-HTP) is shown in Figure 4A. An agent's effect on astrocyte or oligodendrocyte differentiation may also be determined in a similar manner using other cell type specific antibodies.

As described herein, not all cells of a neurosphere are NSCs, and an advantage of the monolayer culture methods of the disclosed invention is the ability to detect the effect of test agents on one or more aspects of NSCs (as opposed to progenitor cells and/or differentiated cells). In some embodiments, monolayer cultures derived from neurospheres can be labeled, for example with antibodies specific for NSCs and/or other cell types (e.g., neurons and glial cells), to determine the proportion of cells in the initial monolayer culture that are NSCs and/or differentiated cells. Similar labeling methods can then be employed after culturing the cells under experimental conditions in the presence of one or more test agents. In this manner, the effect of test agents, as measured by changes in one or more aspects of the cultured cells, can be attributed to changes in the population of NSCs. For example, the proliferation of NSCs can be measured as the number of NSCs produced in culture as a function of the number of NSCs initially plated. Similarly, the number of differentiated cells can be determined relative to the number of NSCs initially plated. Alternatively, the number of differentiated cells measured under experimental conditions can be analyzed relative to the number of differentiated cells in the initial population.

In other embodiments, the aspect(s) of NSCs indicative of neurogenesis can be detected by dissociating cultured neurospheres, and sorting the NSCs, for example by labeling with NSC-specific antibodies in combination with fluorescent-activated cell sorting (FACS). Alternatively, differentiated cells can be sorted by FACS using cell type-specific antibodies, leaving a population of undifferentiated cells. In addition to antibody-based methods, cells can also be labeled by transformation with vectors, such as a plasmid carrying an inducible promoter linked to a reporter construct, as described in more detail below. The sorted NSCs can then be plated for monolayer culture and treated with test agents, for example as described with respect to Examples 4 and 5. Advantageously, the pre-sorting of NSCs prior to performing experiments in monolayer culture enriches the population of cultured cells for NSCs. Such enrichment can allow for detected changes in the properties of the cells to be more accurately attributed to changes in the neurogenic properties of NSCs, and/or reduce the potential for non-NSCs to influence the properties of NSCs in culture.

In some embodiments, the characteristic indicative of the nature and/or degree of neurogenesis is the degree or nature of expression of one or more genes. For example, in some embodiments, the modulation of gene expression is assayed by transforming cultured NSCs with one or more vectors comprising, for example, a promoter of a gene whose expression is indicative of the nature and/or degree of neurogenesis linked to a nucleic acid sequence encoding a reporter construct. The reporter construct may provide a fluorescent, chemiluminescent, chromogenic or bioluminescent signal, such as that provided by green fluorescent protein (GFP), luciferase (luc), yellow fluorescent protein (YFP), and the like. In one embodiment, gene expression is measured using a chemiluminescent or fluorescent substrate detected by Flow Cytometry (FACS analysis) or other automated process. In other embodiments, the reporter construct provides a colorimetric signal detectable by, for example, spectrophotometry, such as that provided by β-galactosidase in the presence of o-nitrophenyl -D-galactopyranoside (ONPG). In particular embodiments, the reporter system allows for the quantitative detection of gene expression. Examples 6 and 7 describe gene reporter assays for use in rodent and human cultured NSCs, respectively.

Several gene-specific promoters have been shown to be specifically activated in rat neural stem cells (rNSC) when the cells differentiate along a neuronal or glial lineage. These promoters include, but are not limited to, promoters specific for the NeuroD1 (ND1), mGluR2, Neurofilament heavy (NFH), GAP43, glial fibrillary acidic protein (GFAP), myelin basic protein (MBP) and nestin genes. As part of the disclsosed invention, these promoters have been confirmed to have a similar predictive function in human neural stem cells (hNSCs).

Modulation of gene expression can also be detected by the production or secretion of one or more polypeptides encoded by a gene. Methods of detecting protein production and/or secretion can include bioassays, binding assays, immunoassays and the like, and are well known to the skilled person.

In some embodiments, the characteristic indicative of the degree and/or nature of neurogenesis is the membrane potential of NSCs. Changes in the membrane potential of NSCs and/or progenitor cells are brought about by ion channels, a class of integral proteins that traverse the cell membrane. There are two types of ion channels in the membrane: gated and nongated. Nongated channels are always open and are not influenced significantly by extrinsic factors. They are primarily important in maintaining the resting membrane potential. Gated channels, in contrast, open and close in response to specific electrical, mechanical, or chemical signals. The charge separation across the membrane, and therefore the resting membrane potential, is disturbed whenever there is a net flux of ions into or out of the cell. A reduction of the charge separation is called depolarization; an increase in charge separation is called hyperpolarization. Changes in the membrane potential of cultured cells can be measured using techniques known to the skilled person.

In some embodiments, the characteristic indicative of the degree and/or nature of neurogenesis is the morphology of NSCs and/or progenitor cells. Cell morphology may be assessed by observing and/or measuring parameters that include, but are not limited to, density, morphology and connectivity of dendritic spines, dendritic arborization, retraction of spines, rate of neurite formation and outgrowth, and other parameters known to the skilled person to correlate with changes in the rate of proliferation, differentiation, migration, and/or survivability of NSCs or progenitor cells. For example, in the hippocampus neural plasticity is believed to underlie changes associated with learning and memory, and can be manifested in the generation of new synapses and the shedding of existing synaptic connections. The sites of synaptic interaction among neurons of the central nervous system are protrusions known as spines that are found on dendritic processes. Dendritic spines are known to change in density, morphology and connectivity in response to a variety of stimuli and are prime candidates as the loci of neural plasticity. In some embodiments, changes in spine density or connectivity in the neurons of the hippocampus may be associated with changes in the capacity for neurogenesis and learning and memory formation. Measurement or detection of these characteristics may be made over the course or, or after, about 21 days or about one month or a longer period.

In the screening assays described herein, a candidate compound that is tested for its ability to modulate neurogenesis can be any type of biological or chemical molecule, including but not limited to, a drug, a small molecule, a peptide, a peptidomimetic, a nucleic acid, a nucleoside analog, such as azidothymidine, dideoxyinosine, dideoxythymidine, dideoxycytidine, or cytosine arabinoside, a carbohydrate, a lipid, a cell such as a stem cell, or any combination thereof. The agent can also include a treatment modality, such as radiation. If desired in a particular assay format, a candidate compound can be detectably labeled or attached to a solid support. In some embodiments, the test agent is a small organic molecule, such as a molecule prepared by combinatorial chemistry methods. In other embodiments, the test agent is a molecule with a molecular weight below about 10kDa, below about 8kDa, below about 6kDa, below about 4kDa, below about 2kDa, or below about 1kDa. In further embodiments, the molecule is capable of, or believed capable of, passing through the blood-brain barrier.

Methods for preparing large libraries of compounds, including simple or complex organic molecules, metal-containing compounds, carbohydrates, peptides, proteins, peptidomimetics, glycoproteins, lipoproteins, nucleic acids, antibodies, and the like, are well known to the skilled person and are described, for example, in Huse, U.S. Pat. No. 5,264,563; Francis et al., Curr. Opin. Chem. Biol. 2:422-428 (1998); Tietze et al., Curr. Biol., 2:363-371 (1998); Sofia, Mol. Divers. 3:75-94 (1998); Eichler et al., Med. Res. Rev. 15:481-496 (1995). Libraries containing large numbers of natural and synthetic compounds for use in methods of the disclosed invention can also be obtained from commercial sources.

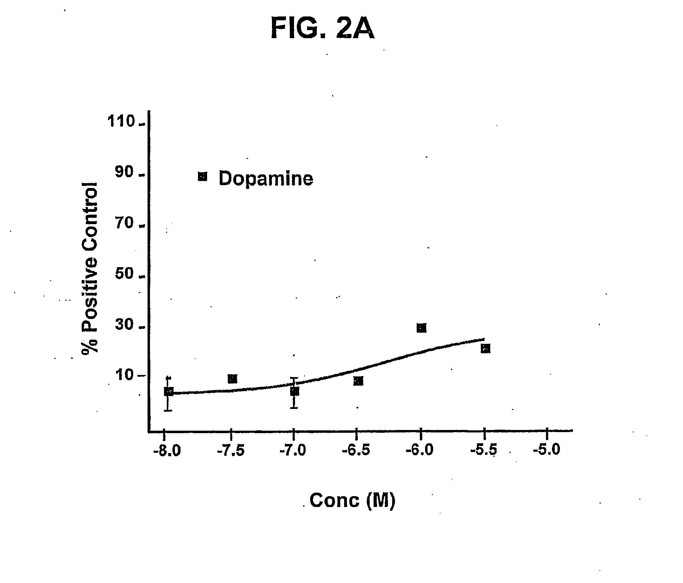

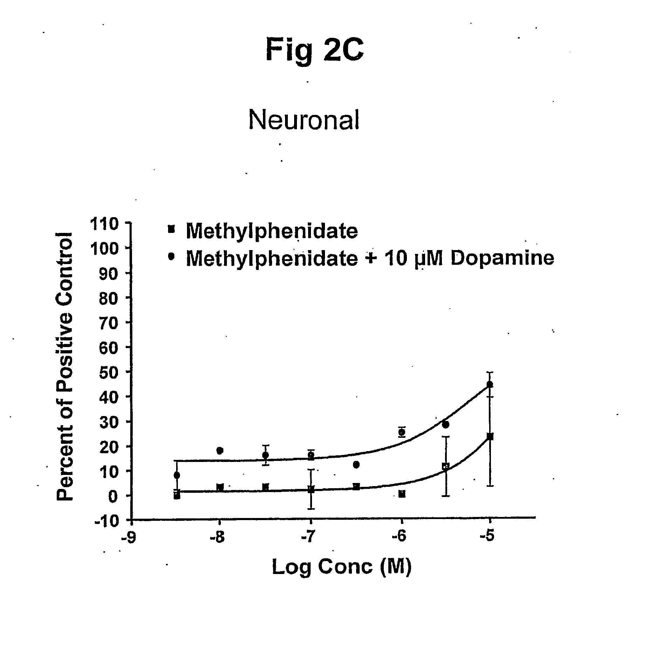

In some embodiments, methods of the disclosed invention are conducted in the presence of one or more factors (hereinafter referred to as "constitutive factors") that facilitate the detection of a neurogenesis modulatory effect of a compound or other treatment modality. The use of neurotransmitters to facilitate detection of the effect of a test agent on the proliferation of human NSCs in monolayer culture is described in Example 8, and is shown in Figures 2-5. Constitutive factors can comprise a single composition or separate compositions, and can be introduced to the test population of neural stem cells at the same time or at different times relative to the test agent or test condition. The use of constitutive factors in methods provided herein is not limited by their sequence or mode of administration. Constitutive factors useful in methods of the disclosed invention can comprise any molecule or treatment modality, including those that potentiate, antagonize, or otherwise modulate neurogenesis. In various embodiments, constitutive factors have a greater-than-additive effect in combination with a test agent on one or more properties of the test population of neural stem cells. For example, in some embodiments, a constitutive factor exerts a synergistic effect with one or more test agents. In further embodiments, a constitutive factor potentiates a neurogenesis modulating effect of a test agent, and/or a test agent potentiates the effect(s) of a constitutive factor. Methods for assessing synergism, potentiation, and other combined pharmacological effects are known to the skilled person, and described, e.g., in Chou and Talalay, Adv Enzyme Regul., 22:27-55 (1984). While constitutive factors used in methods described herein may have greater-than-additive effects in combination with one or more test agents, no particular type or level of response is required to practice the disclosed methods, so long as the presence of the constitutive factor(s) in some way facilitates detection of one or more neurogenesis modulating agents or neurogenic effects.

In some embodiments, the constitutive factor(s) comprise a compound or other component endogenous to the CNS, or a molecule that stimulates and/or inhibits one or more physiological effects of such a compound. Advantageously, the treatment of NSCs with one or more constitutive factors renders the NSCs more amenable to the inducement of changes in the degree and/or nature of neurogenesis by a test agent. In some embodiments, culturing NSCs in the presence of constitutive factors facilitates the detection of neurogenesis modulating agents by increasing the signal to noise ratio of methods of the disclosed invention. Without being bound to any particular theory, the constitutive factors may facilitate detection of neurogenesis modulating agents by mimicking the environment of NSCs *in vivo.* Thus, in some embodiments, the constitutive factors are believed to simulate the environment of brain regions where NSCs and/or neurogenesis are known to occur *in vivo.*

Thus the disclosed invention also includes methods wherein the *in vivo* brain milieu, or chemistry, is modeled by inclusion of endogenous factors. While an *in vitro* model often do not fully reproduce an organism or an *in vivo* context, improvements provided by a method disclosed herein include better replication of the endogenous environment by better modeling of the *in vivo* milieu. For example, the NSC monolayer differentiation assay is improved to better mimic the *in vivo* brain environment by the addition of specific agents to the culture conditions. In some embodiments, an added agent may be one or more constitutive factors. Non-limiting examples of the factors include neurotransmitters, such as those that are a (biogenic) amine and those that are not. The modeling of the in vivo milieu allows for the identification of agents that will modulate NSC differentiation under specific *in vivo* conditions that are not otherwise present in *in vitro* experiments. Example 8 includes the exemplary inclusion of serotonin in a method of the disclosure.

In some embodiments, constitutive factor(s) useful in methods provided herein include one or more neurotransmitters, such as a neurotransmitter (optionally a biogenic amine) which is endogenous to the species, the region of the CNS, and/or the tissue(s) from which the neural stem cells used in the described methods are derived. However, methods provided herein are not limited as to the identity of the factor(s), which may comprise any compound or agent that improves the replication of an endogenous environment. The factor(s) need not be all those which are present in the *in vivo* environment but instead may be any one or more compound or agent.

Thus the disclosed invention includes a method for assaying a test compound for neurogenic activity, the method comprising measuring neurogenesis in neural stem cells exposed to a neurotransmitter. In some embodiments, the cells are in an *in vitro* population of cells comprising neural stem cells in the presence of a growth medium comprising a neurotransmitter. The cells are contacted with a test compound before neurogenesis is measured.

In particular embodiments, the constitutive factor is a biogenic amine, such as dopamine, epinephrine, norepinephrine, serotonin, histamine, or a metabolite, prodrug, or analogue thereof. In further embodiments, the biogenic amine is acetylcholine, tyramine, tryptamine, octopamine, β-phenylethylamine, a phenol amine or a polyamine, or another biologically active amine. In various embodiments, biogenic amines can be used as constitutive factors to facilitate detection of any test agent or condition. For example, Figs. 4A and 4B illustrate the use of a biogenic amine (5-HTP) to facilitate detection of a neurogenesis modulating effect of another biogenic amine (dopamine), which stimulates differentiation of neural stem cells along a neuronal lineage. In addition, Figs. 2A-2C illustrate the use of dopamine as a constitutive factor to facilitate detection of neurogenic effects of other, non-biogenic amine compounds, including amphetamine (Fig. 2B) and methylphenidate (Fig. 2C).

In various embodiments, biogenic amines other than dopamine are used as constitutive factors. For example, in some embodiments, the constitutive factor is serotonin, norepinephrine, histamine, or a metabolite, prodrug, or analogue thereof. For example, Figs. 4A and 4B illustrate the use of the serotonin prodrug 5-hydroxy-tryptophan (5-HTP), which is rapidly metabolized *in vivo* to form serotonin, as a constitutive factor to facilitate the detection of a neurogenesis modulating effect of a neurotransmitter test agent (dopamine). In other embodiments, the presence of a constitutive factor results in a leftward shift of the dose-response curve of a test agent relative to that obtained with the test agent by itself. For example, as shown in Fig. 4B, the addition of a constitutive factor (e.g., 5-HTP) can modulate the IC₅₀ or EC₅₀ value of a test agent. Advantageously, assaying the test agent in the presence of a constitutive factor allows for the detection of neurogenesis modulating effects that would be otherwise undetectable in the absence of the constitutive factor. For example, and without being bound by theory, it is believed that many compounds exert toxic effects at higher doses (e.g., at doses greater than about 5, 15, or 30 µM) that interfere with and/or offset one or more properties measured in the assays described herein. Thus the example shown in Fig. 4B is exemplary of additional embodiments wherein the effect of two or more agents, two or more conditions, or a combination of agent and condition are assayed as disclosed herein to identify their effect(s) in combination. These methods include embodiments wherein one agent is a "constitutive factor" as described herein and another agent, or condition, is a "test agent" or "test condition", respectively.

In further embodiments, norepinephrine is used as a constitutive factor. The effect of norepinephrine on the differentiation of neural stem cells along neuronal and astrocyte lineages is illustrated in Figs. 3A (neuronal) and 3B (astrocyte). In light of these and other teachings disclosed herein, skilled artisans performing routine experimentation can readily utilize norepinephrine and/or other biogenic amines as constitutive factors in the methods described herein.

In some embodiments, the biogenic amine used as a constitutive factor is a "trace amine" (TA), or a metabolite, precursor, prodrug, or analogue thereof. TAs are endogenous, CNS-active amines that are structurally related to classical biogenic amines (e.g., dopamine, 5-HT, norepinephrine). Certain food products, e.g., chocolates, cheeses, and wines, can also provide a significant dietary source of TAs and/or TA-related compounds. Examples of mammalian TAs useful as constitutive factors include, but are not limited to, tryptamine, ρ-tyramine, m-tyramine, octopamine, synephrine and β-phenylethylamine (β-PEA). Additional useful TA-related compounds include, but are not limited to, 5-hydroxytryptamine, amphetamine, bufotenin, 5-methoxytryptamine, dihydromethoxytryptamine, and phenylephrine.

TAs have been shown to bind to and activate a number of unique receptors, termed trace amine-associated receptors (TAARs), which comprise a family of G-protein coupled receptors (TAAR1-TAAR9) with homology to classical biogenic amine receptors. For example, TAAR1 is activated by both tyramine and β-PEA. However, most TAARs have yet to be associated with a specific ligand, suggesting the existence of additional endogenous TAs or TA-related ligands. In addition, binding studies suggest that known TAs bind to non-TAAR sites in the CNS, suggesting other TA receptors and/or pathways. Thus, in various embodiments, the constitutive factor is a ligand of a TAAR, and/or an agent that mediates one or more biological effects of a TA.

In some embodiments, the constitutive factor is β-PEA, which has been indicated as having a significant neuromodulatory role in the mammalian CNS and is found at relatively high levels in the hippocampus (e.g., Taga et al., Biomed Chromatogr., 3(3): 118-20 (1989)). According to the "PEA hypothesis," decreased levels of β-PEA lead to depression, whereas excessive β-PEA levels lead to manic episodes. Without being bound by a particular theory, it is believed that impaired neurogenesis is a significant factor in the etiology of depression, and β-PEA may therefore be required for sufficient levels of neurogenesis, or may otherwise facilitate or modulate neurogenesis. Thus, in various embodiments, β-PEA is used as a constitutive factor to enhance detection of agents that stimulate neurogenesis, and/or agents useful in treating depression. In further embodiments, the constitutive factor is a metabolite, prodrug, precursor, or other analogue of β-PEA, such as the β-PEA precursor L-phenylalanine, which has been shown along with β-PEA to be effective in treating depression; the β-PEA metabolite β-phenylacetic acid (β-PAA), which has been indicated as playing a role in the positive effects of exercise on depressive symptoms; or the β-PEA analogues methylphenidate, amphetamine, and related compounds, which are used to treat cognitive disorders, such ADHD.

Most TAs have a short half-life (e.g., less than about 30 s) due, e.g., to their rapid extracellular metabolism by MAO-A and/or MAO-B, which provide the major pathway for TA metabolism. Thus, in some embodiments, TA levels are regulated by modulating the activity of MAO-A and/or MAO-B. For example, in some embodiments, endogenous TA levels are increased (and TA signaling is enhanced) by administering an inhibitor of MAO-A and/or MAO-B, examples of which are provided herein. TAs have also been shown to have neuromodulatory effects with respect to dopamine, norepinephrine, and 5-HT signaling pathways, for example by inhibiting reuptake by biogenic amine transporters. Thus, in some embodiments, TAs are used as biogenic amine modulators, as more fully described herein.

In additional embodiments, the constitutive factor is a compound, agent, or condition that modulates the levels or activity of a biogenic amine (a "biogenic amine modulator"). For example, in some embodiments, the biogenic amine modulator is an "uptake inhibitor," which increases extracellular levels of one or more monoamine neurotransmitters by inhibiting their transport away from the synaptic cleft and/or other extracellular regions. The term "uptake inhibitors" includes compounds that inhibit the transport of biogenic amines (e.g., uptake inhibitors) and/or the binding of biogenic amine substrates (e.g., uptake blockers) by transporter proteins (e.g., the dopamine transporter (DAT), the NE transporter (NET), the 5-HT transporter (SERT), and/or the extraneuronal monoamine transporter (EMT)) and/or other molecules that mediate the removal of extracellular biogenic amines. For example, Figs. 2B and 2C illustrate the use of amphetamine and methylphenidate, respectively, as constitutive factors. These and other psychostimulants are known to potently inhibit the transport of biogenic amines, which increases their levels in the synaptic cleft, allowing them to facilitate neurogenic effects in various assays provided herein. Biogenic amine uptake inhibitors are generally classified according to their potencies with respect to particular biogenic amines, as described, e.g., in Koe, J. Pharmacol. Exp. Ther. 199: 649-661 (1976). However, references to compounds as being active against one or more biogenic amines are not intended to be exhaustive or inclusive of the monoamines modulated *in vivo,* but rather as general guidance for the skilled practitioner in selecting compounds for use in methods provided herein.

In some embodiments, the biogenic amine modulator is an uptake inhibitor, which may selectively/preferentially inhibit uptake of one or more biogenic amines relative to one or more other biogenic amines. In various embodiments, biogenic amine uptake inhibitors useful in combinations provided herein include, (i) selective serotonin reuptake inhibitors (SSRIs), such as paroxetine (described, e.g., in 3,912,743 and 4,007,196), nefazodone (described, e.g., in 4,338,317), fluoxetine (described, e.g., in 4,314,081 and 4,194,009), sertaline (described, e.g., in 4,536,518), escitalopram (described, e.g., in4,136,193), citalopram (described, e.g., in 4,136,193), fluvoxamine (described, e.g., in 4,085,225), and alaproclate; (ii) serotonin and norepinephrine reuptake inhibitors (SNRIs), such as venlafaxine (described, e.g., in 4,761,501), duloxetine (described, e.g., in 4,956,388), milnacipran (described, e.g., in 4,478,836), sibutramine (BTS 54 524) (described, e.g., in Buckett et al., Prog. Neuro-psychopharmacol. Biol. Psychiatry 12: 575-584 (1988)) and its primary amine metabolite (BTS 54 505), amoxapine, maprotiline, and the tricyclic antidepressants amitriptyline, desipramine (described, e.g., in 3,454,554), and imipramine; (iii) norepinephrine reuptake inhibitors, such as talsupram, tomoxetine, nortriptyline, nisoxetine, reboxetine (described, e.g., in 4,229,449), and tomoxetine (described, e.g., in 4,314,081); (iv) norepinephrine and dopamine reuptake inhibitors, such as bupropion (described, e.g., in 3,819,706 and 3,885,046), and (S,S)-hydroxybupropion (described, e.g., in 6,342,496); and (v) selective dopamine reuptake inhibitors, such as medifoxamine, amineptine (described, e.g., in 3,758,528 and 3,821,249), GBR12909, GBR12783 and GBR13069, described in Andersen, Eur J Pharmacol, 166:493-504 (1989).

In some embodiments, the biogenic amine modulator is a biogenic amine "releaser," which stimulates the release of biogenic amines from presynaptic sites, e.g., by modulating presynaptic receptors (e.g., autoreceptors, heteroreceptors), modulating the packaging (e.g., vesicular formation) and/or release (e.g., vesicular fusion and release) of biogenic amines, and/or otherwise modulating biogenic amine release. Advantageously, biogenic amine releasers provide a method for increasing levels of one or more biogenic amines within the synaptic cleft or other extracellular regions independently of the activity of the presynaptic neuron. Biogenic amine releasers useful in combinations provided herein include, e.g., the 5-HT-releasing agents fenfluramine and p-chloroamphetamine (PCA); and the dopamine, norepinephrine, and serotonin releasing compound amineptine (described, e.g., in 3,758,528 and 3,821,249).

In some embodiments, the biogenic amine modulator is a biogenic amine "metabolic modulator," which increases the extracellular concentration of one or more biogenic amines by inhibiting their metabolism. For example, in some embodiments, the metabolic modulator is an inhibitor of the enzyme monoamine oxidase (MAO), which catalyzes the extracellular breakdown of biogenic amines into inactive species. MAO inhibitors useful in methods provided herein include inhibitors of the MAO-A isoform, which preferentially deaminates 5-hydroxytryptamine (serotonin) (5-HT) and norepinephrine (NE), and/or the MAO-B isoform, which preferentially deaminates phenylethylamine (PEA) and benzylamine (both MAO-A and MAO-B metabolize Dopamine (DA)). In various embodiments, MAO inhibitors may be irreversible or reversible (e.g., reversible inhibitors of MAO-A (RIMA)), and may have varying potencies against MAO-A and/or MAO-B (e.g., non-selective dual inhibitors or isoform-selective inhibitors). Examples of MAO inhibitors useful in methods described herein include clorgyline, L-deprenyl, isocarboxazid (Marplan), ayahuasca, nialamide, iproniazide, iproclozide, moclobemide (Aurorix), phenelzine (Nardil), tranylcypromine (Parnate) (the congeneric of phenelzine), toloxatone, levo-deprenyl (Selegiline), harmala, RIMAs (e.g., moclobemide, described in Da Prada et al., J Pharmacol Exp Ther 248: 400-414 (1989); brofaromine; and befloxatone, described in Curet et al., J Affect Disord 51: 287-303 (1998)), lazabemide (Ro 19 6327), described in Ann. Neurol., 40(1): 99-107 (1996), and SL25.1131, described in Aubin et al., J. Pharmacol. Exp. Ther., 310:1171-1182 (2004).

In some embodiments, the biogenic amine modulator modulates the activity of a biogenic amine receptor, e.g., a serotonin receptor (e.g., 5-HT₁₋₇ receptors), a dopamine receptor (e.g., D₁-D₅ receptors), and/or an adrenergic receptor (e.g., alpha and beta adrenergic receptors). Biogenic amine receptor modulators include compounds that act via any mechanism of action. Examples of receptor modulators include 5-HT_{1A} agonists or partial agonists, such as 8-hydroxy-2-dipropylaminotetralin (8-OHDPAT), buspirone, gepirone, ipsapirone, and flesinoxan; 5-HT_{1A} antagonists, such as WAY 100,635; 5-HT_{2c} agonists or partial agonists, such as *m*-chlorophenylpiperazine; 5-HT_{2A/2c} antagonists, such as ritanserin, etoperidone and nefazodone; dopamine receptor agonists, such as 7-OHDPAT and quinpirole; dopamine receptor antagonists, such as haloperidole, U-99194A, and clozapine; adrenergic antagonists, such as idazoxan and fluparoxan; adrenergic agonists, such as modafanil, salbutamol, clenbuterol, adrafinil, and SR58611A (described in Simiand et al., Eur J Pharmacol, 219:193-201 (1992); and atypical antipsychotics, such as clozapine (Clozaril), olanzapine (Zyprexa), quetiapine (Seroquel), risperidone (Risperdal), ziprasidone (Geodon), aripiprazole (Abilify), and sertindole (Serlect). Additional CNS-active monoamine receptor modulators are well known to the skilled person, and are described, e.g., in the Merck Index, 12th Ed. (1996).

Additional biogenic amine modulators useful in combinations provided herein include tricyclic antidepressants, such as amoxapine, clomiprimine, dothiepen, doxepin, lofepramine (described, e.g., in 4,172,074), trimipramine, and protriptyline; tetracyclic antidepressants, such as mirtazapine (described, e.g., in 4,062,848), mianserin (described, e.g., in 3,534,041), maprotiline (described, e.g., in 3,399,201), and setiptiline; atypical antipsychotics, such as clozapine, olanzapine quetiapine, risperidone, ziprasidone, aripiprazole, and sertindole; and trazodone.

In further embodiments, the constitutive factor(s) utilized in methods of the disclosed invention comprise one or more factors endogenous to the dentate gyrus (DG) region of the hippocampus, where neurogenesis is known to occur in the adult brain. For example, the primary neurons of the dentate gyrus are the granule cells, which receive afferent input from the stellate cells of the entorhinal cortex, the axons of which form the perforant path input to the DG. The DG neurons in turn project to the field CA3 via a bundle of axons known as the mossy fibers. Axons located in the perforant path release the neurotransmitter glutamate, which acts at NMDA receptors, AMPA receptors, and other receptor subtypes. Thus, in some embodiments, the constitutive factor(s) utilized in methods of the disclosed invention include NMDA receptor modulators, such as *N*-methyl-D-aspartic acid (NMDA), which is a non-endogenous amino acid derivative that specifically agonizes NMDA receptors, or NMDA receptor modulator, such as AP5 (2-amino-5phosphonopentanoic acid), DTG, (+)-pentazocine, DHEA, Lu 28-179, BD 1008, ACEA1021, GV150526A, sertraline, or clorgyline. In some embodiments, the constitutive factor(s) may comprise an AMPA receptor agonist, such as alpha-amino-3-hydroxy-5-methyl-4-isoxazolepropionic acid (AMPA), or an AMPA receptor antagonist, such as 7-sulphamoylbenzo-(f)-quinoxaline-2,3-dione (NBQX). Other factors that agonize or antagonize NMDA and/or AMPA receptors are known to the skilled person and may be utilized as constitutive factors.

Another important neurogenic region is the subventricular zone (SVZ) of the lateral ventricles, which is thought to comprise the primary site of neurogenesis in the adult mammalian brain. Neuronal stem and/or progenitor cells originating in the SVZ migrate along the rostral migratory stream (RMS) to the olfactory bulb. Some neural cells undergo various stages of neurogenesis along the RMS, such as migration, proliferation, and various degrees of differentiation. Thus, in some embodiments, the constitutive factor(s) utilized in methods of the disclosed invention comprise one or more factors endogenous to the SVZ, the RMS, and/or the olfactory bulb. A variety of molecules are present in these regions that could potentially influence neurogenesis. For example, γ-aminobutyric acid (GABA) is an inhibitory neurotransmitter found in cultured progenitor cells of the SVZ/RMS that has been associated with various neurological diseases/conditions, including Parkinson's disease and epilepsy. In some embodiments, the constitutive factor is GABA or a molecule that mimics and/or modulates the effect of GABA, such as baclofen or a compound described in Provisional App. 60/731,937. In further embodiments, other neurotransmitters, growth factors, hormones, or other molecules endogenous to the SVZ, RMS, or olfactory bulb are used as constitutive factors.

Additional non-limiting examples of a constitutive factor include one or more growth factors, including but not limited to, LIF, EGF, FGF, bFGF and VEGF. In yet further embodiments, the constitutive factor(s) include one or more ions, which are preferably present at physiologically relevant concentrations. For example, calcium plays an important role in various signaling pathways of the CNS, and sodium is vital in maintaining the resting membrane potential of neurons. In addition, magnesium and other ions can serve as co-factors or modulate the function of other receptor subtypes. Chloride ions also mediate the effects of some receptors, such as GABA receptors. In other embodiments, the constitutive factor(s) comprise a molecule that mimics the effect of an ion or a change in the intracellular or extracellular concentration of an ion.

In some embodiments, the constitutive factors are associated with a physiological state known to facilitate or inhibit neurogenesis, such as stress, aging, exercise, and neural disease/damage. For example, corticosteroids are hormones released by the adrenal glands in response to stress that have been shown to effect neurogenesis in the developing and adult dentate gyrus. Thus, in one aspect, a corticosteroid, such as corticosterone or cortisol, comprises a constitutive factor useful in methods of the disclosed invention. Additional embodiments include the modeling of an *in vivo* disease state as described further below.

In further embodiments, the constitutive factor(s) may be an exogenously supplied factor that might be present *in vivo.* Non-limiting examples include a metabotropic glutamate (mGlu) receptor modulator, such as the compounds provided in the US Prov. App. filed on December 1.4, 2005, to Barlow, entitled, "Methods of Treating Conditions of the Central and Peripheral Nervous Systems by Modulating Neurogenesis"; a muscarinic agent, such as sabcomeline or a compound described in Provisional App. No. 60/727,127; a histone deacetylase modulator, such as valproic acid, MS-275, apicidin, or a compound described in Provisional Application No. 60/715,219; a sigma receptor modulator, such as DTG, pentazocine, SPD-473, or a compound described in Provisional Application No. 60/719,282; a GSK3-beta modulator, such as TDZD-8 or a compound described in Provisional Application No. 60/721,303; a steroid antagonist or partial agonist, such as tamoxifen, cenchroman, clomiphene, droloxifene, or raloxifene; or a phosphodiesterase inhibitor, such as Ibudilast, or a compound described in Provisional App. 60/729,966. Molecules that mimic and/or modulate the physiological effects of one or more neuromodulators, neurotransmitters, or growth factors may also comprise constitutive factors in methods of the disclosed invention.

In some embodiments, the exogenously supplied constitutive factor is a nootropic compound. For example, Fig. 5 illustrates the use of a synthetic nootropic compound (M6 or cyclo-(Pro-Gly)) to facilitate detection of a modulatory effect of the CNS receptor ligand alpha-amino-3-hydroxy-5-methyl-4-isoxazolepropionic acid (AMPA) on the differentiation of neural stem cells along a neuronal lineage. Additional nootropic compounds are known to the skilled person, including but not limited to, piracetam, leviteracetam, nefiracetam, aniracetam, oxiracetam, pyramiracetam, pyritinol, ergot alkaloids, galantamine, selegiline, centrophenoxine, desmopressin, vinpocetine, picamilon, milacemide, and nicergoline. The example shown in Fig. 5 is exemplary of additional embodiments wherein the effect of two or more agents are assayed as disclosed herein to identify their effect(s) in combination. In Fig. 5, AMPA may be considered a "constitutive factor" as described herein while the nootropic compound is a "test agent" which potentiates AMPA activity. Alternatively, and in an equally valid manner as disclosed herein, AMPA may be considered a "test agent" while the nootropic compound is the "constitutive factor" potentiated by AMPA. Embodiments thus include methods to detect or identify a second, or further additional, agent as increasing, potentiating, facilitating, or supporting neurogenesis in combination with a first agent or condition above that seen with the first, second, or further agent alone.

In further embodiments, the exogenously supplied constitutive factor is a non-steroidal anti-inflammatory drugs (NSAIDs), such as celecoxib, rofecoxib, meloxicam, piroxicam, valdecoxib, parecoxib, etoricoxib, etodolac, nimesulide, acemetacin, bufexamac, diflunisal, ethenzamide, etofenamate, flobufen, isoxicam, kebuzone, lonazolac, meclofenamic acid, metamizol, mofebutazone, niflumic acid, oxyphenbutazone, paracetamol, phenidine, propacetamol, propyphenazone, salicylamide, tenoxicam, tiaprofenic acid, oxaprozin, lornoxicam, nabumetone, aspirin, minocycline, benorylate, aloxiprin, salsalate, ibuprofen, naproxen, flurbiprofen, ketoprofen, fenoprofen, fenbufen, benoxaprofen, suprofen, piroxicam, meloxicam, diclofenac, ketorolac, fericlofenac, indomethacin, sulindac, tolmetin, xyphenbutazone, phenylbutazone, feprazone, azapropazone, flufenamic acid or mefenamic acid.

In some embodiments, the exogenously supplied constitutive factor is an opioid receptor antagonist (e.g., mu, delta, and/or kappa antagonists), such as alvimopan, cyprodime (described, e.g., in WO 93/02707), naltrexone (described, e.g., in 3,332,950), naloxone (described, e.g., in 3,254,088), nalmefene (described, e.g., in 3,814,768 and 3,896,226), naltrindole (NTI) (described, e.g., in 4,816,586), nalorphine (described, e.g., in 2,364,833 and 2,891,954), naltriben (NTB) (described, e.g., in 4,816,586), DPI-2505 (described, e.g., in 5,658,908), methiodide, naloxonazine, nalide, nalmexone, nalorphine dinicotinate, naltrindole isothiocyanate, nor-binaltorphimine (nor-BNI), b-funaltrexamine (b-FNA), cyclazocine, methiodide, BNTX, ICI-174,864, LY117413, MR2266 or a compound disclosed in 4,816,586, 4,891,379, 4,191,771, 6,313,312, 6,503,905, or 6,444,679.

In additional embodiments, methods of the disclosed invention are used to measure the ability of one or more test agents to serve a protective function against agents or stimuli known to inhibit neurogenesis. Thus, in some embodiments, the constitutive factor(s) include, but are not limited to, radiation, agents used for chemotherapy, and drugs of abuse, and methods of the disclosed invention are used to detect the ability of one or more test agents or treatment modalities to protect NSCs against the neurogenesis inhibitory effects of the constitutive factor(s). Thus the disclosed invention includes a method of detecting a reduction in toxicity which inhibits or decreases neurogenesis. The method may comprise eXposing a first monolayer cell culture of human neural cells to an agent or condition that inhibits neurogenesis and a second monolayer cell culture of human neural cells to a test agent or condition and said agent or condition that inhibits neurogenesis; and measuring the reduction in toxicity against neurogenesis in said second monolayer in comparison to said first monolayer. In additional embodiments, the method may further compiise identifying an agent or condition that reduces toxicity against neurogenesis as a neuroprotective agent or condition. In some embodiments, the method may be to detect or identify neuroprotective agents or conditions in light of agents such as inflammatory cytokines and astrocyte medicated toxicity.

In yet additional embodiments, methods to detect "toxic" agents, or "toxicity,", are also disclosed. These methods detect or identify agents or conditions that inhibit or decrease neurogenesis via toxicity to cells capable of neurogenesis. A toxicity assay method comprises exposing NSCs to a test agent or condition in the absence of mitogens to identify the agent or condition as being trophic or toxic to the cells. Optionally, the NSCs are dissociate from one or more neurospheres followed by plating with deprivation of mitogens. Alternatively, the NSCs are those of a passaged monolayer from which mitogens have been removed from the medium. Example 9 describes the identification of an exemplary toxic agent like BAY-60-7550.

The disclosed invention further includes methods based on the modeling of an *in vivo* disease condition, or state, by the use of agents or conditions that replicate the condition. These *in vitro* methods allow the identification of agents that are useful for treating disease. As a non-limiting example, opioid-induced depression is a disease state that can result from chronic exposure to opioids. A method to model this disease state using differentiation of monolayer cultures of NSCs, such as human NSCs, is disclosed herein based upon exposure of the cells to an opioid to model the disease state. Agents or conditions that ameliorate, or reverse, that state may then be detected by use of the assay methods disclosed herein.

In some embodiments, the disease condition or state is modeled by the inclusion of inflammatory cytokines, bacterial toxins, or other agents that produce an inflammatory response *in vivo.* Additional examples include components released by reactive astrocytes, such as angiotensin or angiotensin precursors. A method comprising the presence of one or more such agents may be used as a screening tool to identify or detect compounds or conditions that reverse or ameliorate the negative effect(s) of the agent(s) on neurogenesis.

An alternative model for a disease state is present with the detection or measurement of the production of astrocytes, or astrogenesis. Astrocytes are known to be toxic to neurons, and many diseases and conditions are caused or exacerbated by proliferation and/or infiltration of astrocytes into damaged areas of the brain. Non-limiting examples include stroke and other forms of brain injury. Thus additional embodiments of the disclosed invention include a method for the detection or identification of agents and/or conditions that inhibit differentiation of NSCs into astrocytes as well as analogous methods for detecting or identifying agents and/or conditions that increase differentiation into astrocytes. Example 10 describes exemplary methods of the modeling of an *in vivo* disease state.

In various embodiments, methods of the disclosed invention involve comparison to a control, such as those described in the Examples, below. For example, some embodiments include a step of comparing a characteristic of NSCs treated with a test agent to the same characteristic of control NSCs, such as NSCs that have been cultured in parallel to the test cells but have not been administered the test agent. However, comparison to a control is not necessary in methods of the disclosed invention. For example, in some embodiments, the behavior or characteristics of NSCs have been previously characterized under particular conditions, making comparison to a control unnecessary. Where a control is utilized, any type of control can be used that facilitates detection of a neurogenesis modulatory effect or other effect or result of interest. For example, in some embodiments, a control is a preparation or organism that is treated identically to the test preparation, except the control is not exposed to the candidate compound. Another type of control is a preparation that is similar to the test preparation, except that the control preparation is modified so as to be non-responsive to the test compound's neurogenesis modulating effects, such as a cell that does not express a receptor agonized or antagonized by the test compound. In the latter case, the response of the test preparation to a test compound is compared to the response (or lack of response) of the control preparation to the same compound under substantially the same conditions.

In some embodiments, a compound or other treatment modality that modulates neurogenesis will generally promote neurogenesis by at least about 5%, or at least about 10%, about 25%, about 50%, about 100%, about 500% or more, or alternatively reduce neurogenesis by at least about 5%, or about 10%, about 25%, about 50%, about 90% or more, in comparison to a control compound or control condition in the absence of the compound or treatment modality. However, methods of the disclosed invention are not limited to the detections of such changes, but rather can detect any change in the nature, degree, or other aspect of neurogenesis. For example, in some embodiments, methods of the disclosed invention are used to detect the ability of a test agent to protect NSCs from the effects of another agent(s).

Additional aspects of the disclosed invention include methods for identifying populations of cells comprising NSCs and/or progenitor cells for transplantation *in vivo* for experimental, therapeutic, or other purposes. In some embodiments, methods of the disclosed invention are used to detect particular populations of cells, such as those from a particular tissue, host (e.g., from a host diagnosed with a neurological condition), species, cell line, or other source, as having one or more characteristics desirable for transplantation. Characteristics desirable for transplantation can include, for example, the ability of a test agent or treatment modality to modulate the proliferation, differentiation, migration, survival, and/or viability of the cells, as well as the resistance of cells to the effects of a test agent or treatment modality on proliferation, differentiation, migration, survival, and/or viability of the cells. Some disclosed methods are used to identify cell populations that respond to or are resistant to a test agent or other treatment modality in the presence of one or more constitutive factors.

In some embodiments, a method of identifying neural stem cells as suitable for transplantation is disclosed. The method may comprise isolating a subpopulation of neural stem cells from a population of neural stem cells; exposing the subpopulation of cells to an agent or condition which increases neurogenesis; and detecting an increase in neurogenesis in said subpopulation, wherein an increase in neurogenesis indicates that the population of neural stem cells are suitable for transplantation. The increase in neurogeneis may be indicated by an increase in the proportion of neural stem cells, in the subpopulation, that differentiate along a neuronal lineage or a glial lineage. Alternatively, the increase in neurogenesis is indicated by an increase in the proportion of mitotic cells or by an increase in the number of neural stem cells.

In further embodiments, a method of identifying neural stem cells as suitable for transplantation may comprise isolating a subpopulation of neural stem cells from a population of neural stem cells; exposing the subpopulation of cells to an agent or condition' which increases neurogenesis; and detecting the expression of one or more genes in said subpopulation that indicated the presence of neurogenesis, wherein the expression indicates that neural stem cells from the population are suitable for transplantation.

In some embodiments, *in vivo* methods are used to confirm and/or elucidate a neurogenesis modulating effect of a test agent detected using the cell culture techniques detailed above. Advantageously, *in vivo* methods allow compounds to be tested for their effect on neurogenesis both in normal subjects and in subjects having neural damage and disease. Either human subjects or experimental animal models can be used. For example, experimental animal models of trauma due to stroke or neural injury are known to the skilled person. *In vivo* assays that measure the ability of a test agent to modulate neurogenesis can also provide evidence of safety, toxicity, pharmacokinetics and therapeutic efficacy of the compound of interest in preparation for human therapeutic use.

One such *in vivo* technique involves treating cultured NSCs with one or more agents found to modulate neurogenesis, and administering the NSCs to a test animal. In some embodiments, such cells are labeled, for example by transformation with a reporter construct, and the migration, survival, differentiation, or other characteristic of the cells is observed in the test animal.

Because neurogenesis is involved in learning and memory, a neurogenesis modulating effect of a test agent can also be further investigated by administering the test agent to a subject and observing the subject's ability to perform one or more tasks related to cognitive function. Methods for measuring the cognitive functioning of rodents or other mammals are known to the skilled person. In some embodiments, a neurogenesis modulating effect is detected *in vitro* for a test agent using methods of the disclosed invention, and *in vivo* methods are utilized to determine the potential therapeutic use of the agent, for example as an antidepressant, an anti-anxiety medication, or a cognitive enhancer.

Various delivery methods are known to the skilled person and can be used to deliver a test agent to NSCs or progenitor cells within a tissue of interest. The delivery method will depend on factors such as the tissue of interest, the nature of the compound (i.e. its stability and ability to cross the blood-brain barrier), and the duration of the experiment. For example, an osmotic minipump can be implanted into a neurogenic region, such as the lateral ventricle. Alternatively, compounds can be administered by direct injection into the cerebrospinal fluid of the brain or spinal column, or into the eye. Compounds can also be administered into the periphery (such as by intravenous or subcutaneous injection, or oral delivery), and subsequently cross the blood-brain barrier.

Compounds that are found to modulate neurogenesis using methods of the disclosed invention can be used directly as therapeutic agents to prevent or treat a variety of disorders of the nervous system in which it is beneficial to promote, inhibit, or otherwise modulate neurogenesis. Compounds identified by methods of the disclosed invention can also be used to promote, inhibit, or otherwise modulate neurogenesis *ex vivo,* such that a cell composition containing neural stem cells, neural progenitor cells, and/or differentiated neural cells can subsequently be administered to an individual to prevent or treat the same indications. Methods of the disclosed invention can also be used to identify agents and/or conditions that produce undesirable effects on neurogenesis, so that such agents and/or conditions can be avoided, for example by patients suffering from a neurological condition associated with decreased neurogenesis.

Nervous system disorders that can be treated with compounds found to modulate neurogenesis by methods of the disclosed invention include, but are not limited to neurodegenerative disorders, such as Parkinson's disease, Alzheimer's disease, Huntington's Chorea, Lou Gehrig's disease, multiple sclerosis, senile dementia, Pick's disease, Parkinsonism dementia syndrome, progressive subcortical gliosis, progressive supranuclear palsy, thalmic degeneration syndrome, and hereditary aphasia. Also included are neural stem cell disorders, neural progenitor disorders, ischemic disorders, neurological traumas and injuries, affective disorders, neuropsychiatric disorders, degenerative diseases of the retina, retinal injury and trauma, learning and memory disorders, schizophrenia and other psychoses, lissencephaly syndrome, depression, bipolar depression, bipolar disorder, anxiety syndromes, anxiety disorders, phobias, stress and related syndromes, cognitive function disorders, aggression, drug and alcohol abuse, obsessive compulsive behavior syndromes, seasonal mood disorder, borderline personality disorder, and cerebral palsy. In further aspects, the disorders of the nervous system treatable with compounds detected with methods of the disclosed invention include, but are not limited to, dementia, epilepsy, injury related to epilepsy, temporal lobe epilepsy, cord injury, brain injury, brain surgery, trauma related brain injury, trauma related to spinal cord injury, brain injury related to cancer treatment, spinal cord injury related to cancer treatment, brain injury related to infection, brain injury related to inflammation, spinal cord injury related to infection, spinal cord injury related to inflammation, brain injury related to environmental toxin, spinal cord injury related to environmental toxin, autism, attention deficit disorders, narcolepsy, sleep disorders, and cognitive disorders. Compounds identified by methods of the disclosed invention can also be used in normal individuals to enhance learning and/or memory or to treat individuals with defects in learning and/or memory, as well as to treat diseases of the of the peripheral nervous system (PNS), including but not limited to, PNS neuropathies (e.g., vascular neuropathies, diabetic neuropathies, amyloid neuropathies, and the like), neuralgias, neoplasms, myelin-related diseases, etc.

Other conditions that can be beneficially treated with compounds that modulate neurogenesis are known to the skilled person (see, for example, U.S. published application 20020106731).

### EXAMPLES

The following examples are offered to illustrate, but not to limit the claimed invention.

### Example 1: Establishment of Neurosphere Culture From Primary Tissue

Tissues of interest are dissected from a subject (e.g., a human embryo) and placed in petri dishes containing ice-cold 0.6% glucose in PBS (Sigma P4417). Dissected pieces are placed into sterile eppendorf tubes and treated with a 0.1% trypsin solution (Worthington Biochem LS003707) for 10-20 minutes at 37° C. The trypsin is then removed followed by incubation with 0.1% trypsin inhibitor (Sigma T6522) for 10 minutes at 37°C. After removal of the trypsin inhibitor, the sample is incubated with DNAase (Sigma D4527) for 10 minutes at 37°C, followed by removal of the DNAase and incubation with passaging medium (30% Hams F12 (Gibco 11765-062), 70% DMEM (Gibco 11965-118), 1% PSA (Gibco- BRL 15420-062), 2% B27 (Gibco-BRL 17504-044), 20 ng/ml EGF and FGF-2 + heparin (5 micrograms/ml), and optionally, 10 ng/ml LIF (Chemicon LIF1010). The tissue is then triturated with a large bore pipette tip (e.g., P1000) followed by a smaller bore pipette tip (e.g., P200), passaging through each tip approximately 20 times, to achieve a single cell suspension. The cells are counted with a hemocytometer and assessed for viability using trypan blue (Sigma).

Cell suspensions are seeded into T25 or T75 flasks at a density of 200K cells/ml. Flasks are fed every 3 or 4 days by removing half the media and replacing it with fresh media, being careful not to disrupt the spheres. Spheres are maintained in passaging media during initial growth. Spheres are passaged by mechanical chopping using a tissue chopper (McIlwain). After approximately 4 weeks, human neurospheres are switched to maintenance media (30% Hams F12 (Gibco 11765-062), 70% DMEM (Gibco 11965-118), 1 % PSA (Gibco- BRL 15420-062), 1% N2 (Gibco-BRL 17502-048), 20 ng/ml EGF (Sigma E9644)). At 15 to 20 weeks, human cultures are grown in maintenance media supplemented with 10 ng/ml LIF 10 (Chemicon LIF1010). Rodent spheres remain in passaging media for long-term culturing.

### Example 2: Automated, High-Throughput Method for Measuring Growth of Human NSCs Comprising Individual Neurospheres

Human neural stem cells (hNSC) are grown as neurospheres in maintenance media + LIF, as described in Example 1. Neurospheres in maintenance media are cultured for exactly three days after manual dissociation involving exactly two chops using a McIlwein Tissue Chopper set for a 200 um chopping separation with a 90° turn in between chops, followed by a third chop with a 45°. This results in a specific size range with approximately 24% of neurospheres having an area between 0.02 mm² and 0.6 mm², allowing plating into multi-well plates (384- or 1536-well).

For example, and on the third day of culture following manual dissociation, the neurospheres are gently agitated to produce a suspension with the neurospheres evenly distributed, and a sterile pipette is used to transfer a small volume (e.g., 10 µl) of solution to/from each well of a clear bottom 384-well plate (e.g., Costar 3712) such that each well contains one or more neurospheres of the 0.02 to 0.6 size range. Maintenance media is then added to bring each well to a fixed volume (e.g., 30 µl), and one or more test agents are added to assigned wells. Test agents are typically tested in quadruplicate, and at a range of concentrations. Control wells include a positive control comprising maintenance media + LIF and a negative control comprising maintenance media + LIF without EGF/bFGF. Plates are incubated at 37°C, 5% CO₂ for a defined period.

As an example of automation in the assay to increase through-put, images of the wells are taken using an IN Cell Analyzer 1000® plate reader and IN Cell Developer Toolbox® software customized to measure the diameter of each neurosphere. In this high-throughput assay, images may be taken as bright field so that entire neurospheres can be captured through the use of the combination of small wells, [capture image of field; pick spheres; measure; repeat over days/weeks] in a multi-well plate (384- or 1536-well), and low magnification (maximum of 2X- 4X maginification). Essentially, the cell analyzer is used to capture an image of the field defined by a well followed by identification and measurement of neurospheres in the field. This may be repeated over a number of days or weeks such that the same neurospheres in each well are measured over time.

Multi-well plates amenable to the necessary focusing (magnification) and bright field light conditions to allow the required imaging, include Costar black sterile tissue culture treated 384-well plates (catalogue number 3712). Multiple measurements can be taken over a defined incubation period. If neurospheres are incubated longer then several days, maintenance media is replaced with fresh solutions. Data are typically expressed as % change over baseline using the equation: [(Area at time 0) - (Area at time X)/(Area at time 0)*100]. The compound tacrine promoted neurosphere growth as seen in Figure 6.

### Example 3: Transfer of Human Neurospheres to Monolayer Culture

Human neural stem cells (hNSC) are grown as neurospheres in maintenance media, as described in Example 1. The cells are routinely passaged every 7-14 days by mechanical chopping on a tissue chopper (McIIlwain Instruments) to a sphere diameter of 200. µm, and are fed every 3 to 4 days by replacing half of the media with fresh media. The neurospheres are transferred to adherent monolayer cultures after dissociation by enzymatic treatment with ACCUTASE™ (a combination of enzymes, phosphate buffered saline, and phenol red from Innovative Cell Technologies, San Diego), or alternatively trypsin (Worthington Biochem LS003707).

Briefly, the neurospheres are transferred to an eppendorf tube, allowed to settle for one minute, and treated with ACCUTASE™ pre-warmed to 37°C for 10 minutes. The neurospheres are dissociated by gentle trituration with a P200 tip approximately 20-30 times. After centrifugation for 2 minutes at 200g, the cells are washed with maintenance media (30% Hams F12 (Gibco 11765-062), 70% DMEM (Gibco 11965-118), 1% PSA (Gibco- BRL 15420-062), 1% N2 (Gibco-BRL 17502-048), 20 ng/ml. EGF (Sigma E9644)) and counted with a hemocytometer and assessed for viability using trypan blue (Sigma). The cells are plated out on surfaces coated with 10 µg/ml poLy-L-lysine (Sigma P5899) and 50 µg/ml mouse Laminin (L2020).

Passaging and long term growth of adherent hNSC was achieved using a medium comprising 30% Hams F12 (Gibco 11765-062), 70% DMEM (Gibco 11965-118), 1% PSA (Gibco- BRL 15420-062), 1% N2 (Gibco-BRL 17502-048), 20 ng/ml EGF (Sigma E9644), 10ng/ml LIF (Chemicon LIF1010), 20ng/ml bFGF (R and D 233-FB) and 5 µg/ml heparin (Sigma H3149). The adherent cells are routinely passaged every 2-3 days by briefly incubating them in warm ACCUTASE™ until the cells lift off, and harvesting the cells by rinsing with maintenance media, followed by centrifugation for 3 min at 1000rpm. The cells are counted and re-plated out on surfaces coated with 10 µg/ml poly-L-lysine and 50 µg/ml mouse Laminin. This permitted stable culturing of human neural stem cells as a monolayer based upon exposure to specific factors in a defined medium, as well as culture on a defined substrate. This also allowed passaging of cells in a monolayer form such that the stem cell nature is preserved.

The ability of the NSCs cultured as monolayers to differentiate into multiple lineages may be confirmed via exposure to different agents that promote specific cell fates as follows. NSCs are obtained and plated in 96-well plates, and treated with test compounds as described above, except that the test media does not contain EGF or bFGF (mitogen-free test media). Alternatively, the initial test media is as described above, but the test media is exchanged after a defined period, for example at day 4, with mitogen-free test media. The following controls are included: Control 1: mitogen-free test media with 10µM DHEA (positive control for neuronal differentiation); Control 2: test media with EGF and bFGF (negative control); Control 3: mitogen-free test media with 50ng/ml BMP-2 and 0.5% FBS (positive control for astrocyte differentiation); and Control 4: mitogen-free test media with 2ng/ml IGF-1 (positive control for oligodendrocyte differentiation).

Plates are incubated, washed, and fixed as described above. Fixed cells are stained with cell type-specific antibodies. Examples of such antibodies include GFAP (astrocytes), TUJ-1 and NF-200 (neurons), and O1 and O4 (oligodendrocytes). The ability to differentiate into these multiple neuronal lineages (cell types) was confirmed (data not shown). This reflects the retention or maintenance of the characteristic feature of NSCs to differentiate in a monolayer culture.

### Example 4: Automated Proliferation (Growth or Trophism) Assay Using Monolayer Cultures

Sterile, tissue culture treated, clear bottom 96-well plates (e.g., Costar 3712) are coated 10 µg/ml poly-L-lysine and 50 µg/ml mouse Laminin, as described in Example 2. 10-15 neurospheres in maintenance media (see, Example 2) are transferred (estimated at 100,000 cells/sphere) to an eppendorf tube and enzymatically dissociated as described in Example 2. Approximately 50,000 cells are plated per 100µl/well. The cells are allowed to attach by incubating for approximately one hour, and 100µl/Vwell of test compounds are added. Figure 1A shows the well assignments for a typical experiment testing 5 compounds in duplicate to obtain 8-point dose response curves. Compounds are prepared using Perkin-Elmer MultiPROBE II PLUS _{HT EX} (Protocol 8-point HumanCRC96-well) in test media (30% Hams F12 (Gibco 11765-062), 70% DMEM (Gibco 11965-118), 1% PSA (Gibco-BRL 15420-062), 1% N2 (Gibco-BRL 17502-048), 20 ng/ml EGF (Sigma E9644), 10ng/ml LIF (Chemicon LIF1010), 20ng/ml bFGF (R and D 233-FB) and 5 µg/ml heparin (Sigma H3149)).

Each plate contains controls containing test media (positive control) and test media without growth factors (negative control). Plates are incubated at 37°C and 5% CO₂ for a total of 7 days. At day 4, the cell plates are aspirated and fresh compound/media is added. After incubation, the wells are washed 1X with 0.1M Tris-Buffered Saline (TBS), and incubated at room temperature for 30 minutes with 100µl/well of Fixing/Nuclear staining solution (8µg/ml Hoechst 33342 and 3.7% Formaldehyde in 0.1M TBS). Cells are then washed 4X with 0.1M TBS. The number of cells per well or per image (i.e. field of view) is measured using an IN Cell Analyzer 1000® plate reader and IN Cell Developer Toolbox® software. A typical dose-response curve for a non-neurogenic (non-tropic) and non-toxic compound (naltrexone) is illustrated in Fig. 1B.

### Example 5: Automated Differentiation Assay Using Monolayer Cultures

The identification of differentiated NSCs and manual counting of the relative numbers of neurons, astrocyte, and other cells in a monolayer as described in Example 3 may be performed in a routine manner. A high-throughput, high content assay to evaluate the effects of different agents upon the differentiation of NSCs is possible by use of stable monolayer NSC culturing methods as described in Example 3 with optional miniaturization of the differentiation system.

Miniaturization of the assay to permit multiple analyses in a concentration response curve format was achieved for human NSCs by plating cells at the specific density of about 78,125 cells/cm² into a high-throughput, multi-well plate, including 96-, 384-, and 1536-well plates, coated with the substrate of 10 µg/ml poly-D-lysine and 50 µg/ml mouse Laminin. Cells were cultured in mitogen free test media or exposed to differentiating agents as described herein immediately upon plating of cells. Stable, differentiation-compatible culture may be used with automated equipment (like Perkin Elmer Evolution P3, Perkin Elmer Multiprobe II Plus and Bio-Tek ELx405 Select CW as non-limiting examples) to replace 50% of media with newly prepared media and differentiation agent between 3 to 4 days after commencement of the experiment.

Measurement of the resulting NSC differentiation may be performed by fixation and staining as described herein and subsequent use of automated equipment (InCell Analyzer high throughput imaging system) to take multiple pictures per well, at multiple wavelengths. Quantification of neuronal differentiation was performed by measuring the amount of Tuj1 staining and dividing the number by the number of cells as determined through automated counting of Hoechst stained cell nuclei. Quantification of astocytic differentiation was performed by measuring the amount of GFAP staining and dividing the number by the number of cells as determined through automated counting of Hoechst stained cell nuclei. A concentration response curve demonstrating increased differentiation of NSCs into neurons with increased concentrations of serotonin (5-HTP) is shown in Figure 4A. An agent's effect on astrocyte or oligodendrocyte differentiation can be determined in a similar manner using other cell type specific antibodies, examples of which include GFAP (astrocytes), NF-200 (neurons), and O1 and 04 (oligodendrocytes).

### Example 6: in vitro Rodent Gene Reporter Assay

Rodent neural stem cells (rNSC) are cultured in maintenance media with bFGF (30% Hams F12 (Gibco 11765-062), 70% DMEM (Gibco 11965-118), 1% PSA (Gibco-BRL 15420-062), 1% N2 (Gibco-BRL 17502-048), 20 ng/ml bFGF (R and D 233-FB), 1 mM L-glutamine). All plastic or glassware is coated with 10 ug/ml poly-L-ornithine and 5 ug/ml mouse Laminin. The cells are isolated by incubation with trypsin at room temperature for 1 minute, resuspension in 5 ml maintenance media, centrifugation at 1000g for 3 min, and resuspension in 1 ml maintenance media with gentle trituration using a small bore Pasteur pipette. The cells are then counted with a hemocytometer and assessed for viability using trypan blue (Sigma). A mix consisting of 0.5 µg renilla luciferase and 5 µg promoter specific sea pansy luciferase is used with gene specific promoters linked to green fluorescent protein (GFP), yellow fluorescent protein (YFP) or the fluorescent protein DsRed. All gene reporter constructs are cloned in the same lentiviral vector backbone.

A GFP vector control is used in parallel to visualize effectiveness of electroporation. 2x10⁶ cells are typically used for each electroporation. The resuspended cells are mixed with the DNA to be transfected in 100 µL of Nucleofactor solution. The mixture is then transferred to an electroporation vial, electroporated, and the cells are mixed with 500 µL of maintenance media. 9.5mL of maintenance media is added per electroporation to an equal volume of maintenance media containing a twofold concentration of the drug to be tested. The cells are incubated in 5% CO₂ at 37°C for 2 days, the media is aspirated and the appropriate amount of lysis buffer is added. The cell extracts are read immediately, or alternatively frozen for later analysis. The promoter-specific activation of luciferase or levels of fluorescent protein are analyzed, for example on a Tecan Genios Pro reader.

### Example 7: Human in vitro gene reporter assay

Human cortical stem cells are grown as neurospheres in maintenance media with EGF/LIF (30% Hams F12 (Gibco 11765-062), 70% DMEM (Gibco 11965-118), 1% PSA (Gibco- BRL 15420-062), 1% N2 (Gibco-BRL 17502-048), 20 ng/ml EGF (Sigma E9644), 20ng/ml LIF (Chemicon LIF1010)). The cells are passaged by chopping into quarters on a tissue chopper every 10-14 days. The sphere diameter preferably does not exceed 500 µm. The cells are fed every 3 to 4 days by taking off half of the old conditioned media and by adding half fresh media. Neurospheres can optionally be passaged as monolayers, as described in Example 3. The cells are transfected with promoter specific gene reporter constructs, and the levels of promoter activation measured as described in Example 6.

### Example 8: Use of Neurotransmitters as Constitutive Factors to Facilitate Detection of Neurogenesis Modulation In Vitro

Human NSCs maintained as neurospheres are plated as monolayers on laminin/ poly-L-lysine coated plates and assayed for proliferation and/or differentiation, as described in Examples 3 and 4, except that the test media is modified to include one or more neurotransmitters at various concentrations to facilitate detection of neurogenesis modulating agents. Figure 4B shows results for a neurotransmitter (serotonin or 5-HTP) that facilitates detection of the effect of a test agent (dopamine) on NSC proliferation. Proliferation is measured as the mean cell intensity per microscopic field of view, with the mean cell intensity for control experiments subtracted. Data is plotted as a dose response curve of dopamine with and without 2 independent concentrations of 5-HTP.

In the example given, each curve shows mean cell intensity as a function of test agent concentration, with the respective background cell intensity levels subtracted (values in media only are subtracted from the dopamine curve, and values in media with 10 µM 5-HTP and 30 µM 5-HTP are subtracted from the corresponding values in the presence of dopamine). The test agent (dopamine) has a small dose-dependent effect on NSC proliferation (squares) that is substantially enhanced in the presence of 10 µM 5-HTP (circles), and further enhanced in the presence of 30 µM 5-HTP (triangles), particularly at higher concentrations of the test agent. The data show a synergistic enhancement of neuronal differentiation by the neurotransmitter and the test agent.

This example shows that the *in vivo* brain milieu may be modeled by using one or more endogenous factors present in the brain. Cells were cultured in the presence of dopamine (a component of brain chemistry *in vivo*) and neuronal differentiation determined as described in Example 5. Dopamine alone did not promote differentiation of NSCs into neurons. However, the addition of a neurotransmitter normally found in the brain, 5-HTP, sensitizes the cells to exposure to dopamine, resulting in a concentration-dependent increase in NSC differentiation in response to dopamine.

### Example 9: Automated NSC Monolayer Toxicity/Trophism Assay

Development of a high-throughput, automated assay to measure trophic effects of agents on NSCs is provided by optimization of monolayer culture conditions as described above, miniaturization of the culturing system as described above.

Briefly, cells were cultured as described in Example 5 with the presence of histamine. The histamine was dissolved in DMSO as a vehicle, and cells were exposed to a maximum concentration of 0.3% vehicle. Cell number was determined by fixation and exposure of cells to Hoechst stain. Image acquisition was automated with the use of an InCell Analyzer 1000, and the number of cells determined by automated counting of stained nuclei. Histamine promoted cell growth in a concentration-dependent manner. (see Figure 8).

Toxic effects of agents can be determined in a similar manner. NSCs were exposed to BAY-60-7550 as described above and cell number was quantified. BAY-60-7550 caused cell death in a concentration-dependent manner, indicating toxicity at higher concentrations (see Figure 9).

### Example 10: Modeling an in vivo disease state

Normal neuronal differentiation levels were created using the endogenously derived factor DHEA. Exposure of these cells simultaneously to the opioid morphine resulted in inhibition of normal NSC differentiation. An assay was developed to identify agents that could restore neurogenesis. Cells were exposed to both morphine and naltrexone (an inhibitor of morphine action). The exposure to naltrexone resulted in rescue of neurogenesis (see Figure 10). Cells were plated, cultured, imaged and analyzed as described above in the monolayer assay.

An assay for astrogenesis can be used to identify agents that inhibit differentiation of NSCs into astrocytes. The 5-HT1a agonist buspirone promotes differentiation into both neurons and astrocytes (see Figure 11). Melatonin alone shows no effect on astrogenesis, but the addition of increasing concentrations of melatonin to the concentraton-response curve of buspirone results in the repression of astrocytes, while differentiation into neurons is preserved. Cells were plated, cultured, imaged and analyzed as described above.

All references cited herein, including patents, patent applications, and publications, are hereby incorporated by reference in their entireties, whether previously specifically incorporated or not.

Having now fully provided the instant disclosure, it will be appreciated by those skilled in the art that the same can be performed within a wide range of equivalent parameters, concentrations, and conditions without departing from the spirit and scope of the disclosure and without undue experimentation.

While the disclosure has been described in connection with specific embodiments thereof, it will be understood that it is capable of further modifications. This application is intended to cover any variations, uses, or adaptations of the disclosure following, in general, the disclosed principles and including such departures from the disclosure as come within known or customary practice within the art to which the disclosure pertains and as may be applied to the essential features hereinbefore set forth.

## Claims

1. A method for identifying an agent or condition that modulates neurogenesis, said method comprising
exposing a monolayer cell culture comprising human neural cells to a test agent or condition, and
identifying said test agent or condition as modulating neurogenesis in said cells after measuring a property indicative of neurogenesis in said cells, wherein said neural cells optionally comprise human neural stem cells (NSCs).

2. The method of claim 1, wherein said exposing, and optionally said identifying, is in the presence of EGF, bFGF, FGF, VEGF, LIF, a monoamine, or a neurotransmitter.

3. The method of claim 1 or 2, wherein said modulating of neurogenesis is indicated by a change in the proportion of neural cells in mitosis.

4. The method of claim 1 or 2, wherein said modulating of neurogenesis is indicated by a change in expression of one or more genes in said neural cells.

5. The method of claim 1 or 2 or 3 or 4, wherein said cell culture comprises NSCs and said modulating of neurogenesis is indicated by a change in the proportion of NSCs in said culture.

6. The method of claim 1 or 2, wherein said modulating of neurogenesis is indicated by a change in the population of neurons or astrocytes in said culture.

7. The method of claim 1 or 2 or 3 or 4 or 5 or 6, wherein said exposing is in the presence of a second agent or condition, wherein the second agent or condition enhances the modulation of neurogenesis in said cell culture.

8. The method of claim 7, wherein the second agent or condition is a monoamine neurotransmitter agent, optionally selected from serotonin, dopamine, norepinephrine, and analogues, metabolites, or prodrugs of any of the foregoing.

9. The method of claim 7, wherein the second agent or condition is an agent that modulates the level or effect of one or more neurotransmitters, or monoamines, such as the reuptake of a monoamine neurotransmitter.

10. The method of claim 7, wherein the second agent or condition is a monoamine receptor modulator or a MAO inhibitor.

11. A method of detecting a reduction in toxicity, said method comprising
exposing a first monolayer cell culture of human neural cells to an agent or condition that inhibits neurogenesis and a second monolayer cell culture of human neural cells to a test agent or condition and said agent or condition that inhibits neurogenesis; and
measuring the reduction in toxicity against neurogenesis in said second monolayer in comparison to said first monolayer.

12. The method of claim 11 further comprising identifying an agent or condition that reduces toxicity against neurogenesis as a neuroprotective agent or condition.

13. The method of claim 8, wherein the neurotransmitter is a monoamine, optionally selected from dopamine, serotonin, or norepinephrine.

14. The method of claim 1, wherein said measuring comprises detecting growth of said neural stem cells.

15. A method for identifying an agent or condition that modulates neurogenesis, the method comprising:
exposing a neurosphere having a cross-sectional area of at least about 0.2 mm² to about 0.6 mm² to a test agent or test condition; and
identifying said test agent or test condition as modulating neurogenesis in said neurosphere after measuring a property indicative of neurogenesis in said cells.
